# FASCICULE DE BREVET EUROPEEN

(11) **EP 3 292 409 B1**
(45) Date de publication et mention de la délivrance du brevet: **29.11.2023**
(21) Numéro de dépôt: 16805165.4
(22) Date de dépôt: 21.10.2016
(51) Int. Cl.: G01N 33/52, C09K 11/06, A61P 35/00, A61P 43/00

(54) **PARTICULE COMPRENANT AU MOINS UNE NANOPARTICULE D'OXYDE DE FER FERRIMAGNÉTIQUE OU FERROMAGNÉTIQUE ASSOCIÉE À AU MOINS UN COMPOSÉ POUR UNE UTILISATION MÉDICALE OU COSMÉTIQUE**
PARTIKEL MIT MINDESTENS EINEM FERRIMAGNETISCHEN ODER FERROMAGNETISCHEN EISENOXIDNANOPARTIKEL IM ZUSAMMENHANG MIT MINDESTENS EINER VERBINDUNG ZUR MEDIZINISCHEN ODER KOSMETISCHEN VERWENDUNG
PARTICLE COMPRISING AT LEAST ONE FERRIMAGNETIC OR FERROMAGNETIC IRON OXIDE NANOPARTICLE ASSOCIATED WITH AT LEAST ONE COMPOUND FOR MEDICAL OR COSMETIC USE

(30) Priorité: 21.10.2015 FR 1502228
(43) Date de publication de la demande: 14.03.2018
(73) Titulaire: Nanobacterie, 75116 Paris (FR)
(72) Inventeur: ALPHANDÉRY, Edouard, 75116 Paris (FR); CHEBBI, Imène, 91120 Palaiseau (FR)
(74) Mandataire: Jorget, Quentin
(86) Numéro de dépôt international: PCT/FR2016/000173
(87) Numéro de publication internationale: WO 2017/068252

(56) Documents cités:
- US-A1- 2014 155 733
- SANTANEEL GHOSH ET AL: "Controlled actuation of alternating magnetic field-sensitive tunable hydrogels;Controlled actuation of alternating magnetic field-sensitive tunable hydrogels", JOURNAL OF PHYSICS D: APPLIED PHYSICS, INSTITUTE OF PHYSICS PUBLISHING LTD, GB, vol. 43, no. 41, 29 septembre 2010 (2010-09-29), page 415504, XP020199120, ISSN: 0022-3727, DOI: 10.1088/0022-3727/43/41/415504
- PURUSHOTHAM S ET AL: "Thermoresponsive core-shell magnetic nanoparticles for combined modalities of cancer therapy", NANOTECHNOLOGY, IOP, BRISTOL, GB, vol. 20, no. 30, 29 juillet 2009 (2009-07-29), page 305101, XP020160740, ISSN: 0957-4484
- TAO TANG ET AL: "Fluorescence imaging and targeted distribution of bacterial magnetic particles in nude mice", APPLIED MICROBIOLOGY AND BIOTECHNOLOGY, SPRINGER, BERLIN, DE, vol. 94, no. 2, 8 mars 2012 (2012-03-08), pages 495-503, XP035033401, ISSN: 1432-0614, DOI: 10.1007/S00253-012-3981-8
- HUANG HENG ET AL: "Local Optical Temperature Measurements around Magnetosomes within Single Bacteria to Study Size and Geometry Effects on Heating", BIOPHYSICAL JOURNAL, vol. 106, no. 2, 17 février 2014 (2014-02-17), XP028823859, ISSN: 0006-3495, DOI: 10.1016/J.BPJ.2013.11.2163
- SAÏWAN BUATHONG ET AL: "Thermal, Magnetic, and Luminescent Properties of Dendronized Ferrite Nanoparticles", JOURNAL OF PHYSICAL CHEMISTRY C, vol. 113, no. 28, 16 juillet 2009 (2009-07-16), pages 12201-12212, XP055275676, US ISSN: 1932-7447, DOI: 10.1021/jp902046d
- C. LANG ET AL: "Expression of Green Fluorescent Protein Fused to Magnetosome Proteins in Microaerophilic Magnetotactic Bacteria", APPLIED AND ENVIRONMENTAL MICROBIOLOGY, vol. 74, no. 15, 6 juin 2008 (2008-06-06), pages 4944-4953, XP055105822, ISSN: 0099-2240, DOI: 10.1128/AEM.00231-08
- FREDDI S ET AL: "A molecular thermometer for nanoparticles for optical hyperthermia", NANO LETTERS, AMERICAN CHEMICAL SOCIETY, US, vol. 13, no. 5, 8 mai 2013 (2013-05-08), pages 2004-2010, XP002724230, ISSN: 1530-6984, DOI: 10.1021/NL400129V [extrait le 2013-04-26]
- Jin Xie ET AL: "Production, Modification and Bio-Applications of Magnetic Nanoparticles Gestated by Magnetotactic Bacteria", , 31 décembre 2009 (2009-12-31), XP055261235, Extrait de l'Internet: URL:http://download.springer.com/static/pd f/73/art%3A10.1007%2Fs12274-009-9025-8.pdf ?originUrl=http://link.springer.com/articl e/10.1007/s12274-009-9025-8&token2=exp=145 9255843~acl=/static/pdf/73/art%253A10.1007 %252Fs12274-009-9025-8.pdf?originUrl=http% 3A%2F%2Flink.springer.com%2Farticle%2F10.1 007%2Fs122 [extrait le 2016-03-29]
- EDOUARD ALPHANDÉRY ET AL: "Nanoprobe Synthesized by Magnetotactic Bacteria, Detecting Fluorescence Variations under Dissociation of Rhodamine B from Magnetosomes following Temperature, pH Changes, or the Application of Radiation", ACS APPLIED MATERIALS & INTERFACES, vol. 9, no. 42, 16 October 2017 (2017-10-16), pages 36561-36572, XP055532920, US ISSN: 1944-8244, DOI: 10.1021/acsami.7b09720
- Kohei Maruyama ET AL: "Single nucleotide polymorphism detection in aldehyde dehydrogenase 2 (ALDH2) gene using bacterial magnetic particles based on dissociation curve analysis", BIOTECHNOLOGY AND BIOENGINEERING, vol. 87, no. 6, 16 August 2004 (2004-08-16), pages 687-694, XP055275589, ISSN: 0006-3592, DOI: 10.1002/bit.20073
- DIMITROV ET AL: "Magnetic properties of superparamagnetic particles by a Monte Carlo method.", PHYSICAL REVIEW. B, CONDENSED MATTER 01 OCT 1996, vol. 54, no. 13, 1 October 1996 (1996-10-01), pages 9237-9241, ISSN: 0163-1829
- GRANITZER PETRA ET AL: "Magnetic properties of superparamagnetic nanoparticles loaded into silicon nanotubes.", NANOSCALE RESEARCH LETTERS 2014, vol. 9, no. 1, 2014, page 413, ISSN: 1931-7573

## Description

### DOMAINE DE L'INVENTION

Le domaine de l'invention concerne des particules comprenant au moins une nanoparticule d'oxyde de fer ferrimagnétique ou ferromagnétique associée à au moins un composé et leur utilisation médicale ou cosmétique.

### ÉTAT DE LA TECHNIQUE

Le fonctionnement de certaines nouvelles sondes repose sur la détection locale d'un paramètre tel que la température. Afin d'y parvenir, des thermomètres fluorescents ont été mis au point contenant des nanoparticules d'oxyde de fer associées à un fluorophore en utilisant des méthodes permettant habituellement d'éviter l'atténuation de luminescence par l'oxyde de fer, (S.K. Mandai et al, Langmuir, Vol. 21, P. 4175 (2005)). Toutefois, ces nanoparticules sont superparamagnétiques et sont donc faiblement magnétiques. Elles possèdent un moment magnétique instable à température physiologique ou ambiante.

De nouveaux modes de délivrance de médicaments ont également été proposés en associant les médicaments à des nanoparticules, notamment à des magnétosomes, permettant d'améliorer l'efficacité des médicaments, (J-B. Sun et al, Cancer Letters, Vol. 258, P. 109 (2007)). Toutefois, le médicament ne peut pas dans ce cas être relargué de manière contrôlée. Santaneel Ghosh and Tong Cai 2010 J. Phys. D: Appl. Phys. 43 415504 "Controlled actuation of alternating magnetic field-sensitive tunable hydrogels; Controlled actuation of alternating magnetic field-sensitive tunable hydrogels" a pour objet un système complexe d'hydrogel magnétique constitué d'un tube capillaire polymérisé avec un polymère thermosensible contenant un grand nombre de nanoparticules ferromagnétiques.

US2014/155733 A1 concerne un procédé et une composition pour le traitement hyperthermique des cellules tumorales chez un patient dans des conditions qui affectent les cellules souches tumorales et les cellules tumorales par administration de nanoparticules ferromagnétiques. Purushotham, S et al. "Thermoresponsive core-shell magnetic nanoparticles for combined modalities of cancer therapy." Nanotechnology vol. 20,30 (2009): 305101. doi: 10.1088/0957-4484/20/30/305101 concerne l'utilisation de nanoparticules magnétiques chargées de médicaments anticancéreux pour le traitement multimodal du cancer.

Tang, Tao et al. "Fluorescence imaging and targeted distribution of bacterial magnetic particles in nude mice." Applied microbiology and biotechnology vol. 94,2 (2012): 495-503. doi:10.1007/s00253-012-3981-8 décrit une méthode de fabrication de magnétosomes rendus fluorescents en les associant à des molécules de Dil. Ces magnétosomes fluorescents sont utilisés pour étudier leur distribution in vivo.

Freddi, Stefano et al. "A molecular thermometer for nanoparticles for optical hyperthermia." Nano letters vol. 13,5 (2013): 2004-10. doi:10.1021/nl400129v concerne un thermomètre moléculaire pour les nanoparticules d'or ou d'oxyde de fer paramagnétique dans l'hyperthermie optique.

### DESCRIPTION DE L'INVENTION

L'objet de la présente invention est un procédé *in vitro* de détection de variation de luminescence, comprenant :
a) une étape dans laquelle une perturbation physico-chimique est exercée sur une particule utilisée à titre de sonde ;
   dans laquelle la perturbation physico-chimique est exercée sur la particule par un rayonnement électromagnétique, de rayons X, un rayonnement dû à un champ magnétique, une variation de pH, et/ou une variation de température ;
   ladite particule comprenant au moins une nanoparticule d'oxyde de fer ferrimagnétique ou ferromagnétique associée à au moins un composé, où la nanoparticule n'est pas liée au composé par au moins un acide nucléique ou par au moins un acide aminé, dans laquelle ledit composé se dissocie de la nanoparticule d'oxyde de fer, où la dissociation du composé n'entraine pas sa destruction, suite à ladite perturbation physico-chimique exercée sur la particule, où la perturbation physico-chimique induit une dissociation de la liaison faible entre ledit composé et ladite nanoparticule, ledit composé étant une substance luminescente qui possède au moins une fonction chimique sélectionnée dans le groupe constitué des fonctions acide carboxylique, phosphorique, sulfonique, ester, amide, cétone, alcool, phénol, thiol, amine, éther, sulfure, anhydride d'acide, halogénure d'acyle, amidine, nitrile, hydropéroxyde, imine, aldéhyde, peroxyde et ayant au-moins une propriété de luminescence qui varie lors de la dissociation entre ladite nanoparticule et ledit composé ; puis
b) une étape de détection de variation de luminescence de ladite particule utilisée à titre de sonde.

La particule selon l'invention de procédé peut comprendre au moins 1, 10, 10², 10, 10⁴, 10⁵, 10⁶, 10⁹, 10²⁰, ou 10⁴⁰ nanoparticule(s) d'oxyde de fer ferrimagnétique ou ferromagnétique et/ou au moins 1, 10, 10², 10³, 10⁴, 10⁵, 10⁶, 10⁹, 10²⁰, ou 10⁴⁰ composé(s).

La particule selon l'invention de procédé, lorsqu'elle est associée à une substance luminescente, diffère dans son fonctionnement des sondes nanoparticulaires luminescentes décrites habituellement. En effet, son mode de fonctionnement original repose sur la détection d'une variation de luminescence due à la dissociation d'une substance luminescente associée à la particule, suite à une perturbation physico-chimique exercée sur la particule.

Dans un mode de réalisation, la luminescence est associée à au moins une propriété de luminescence qui varie de moins de 10⁻¹⁰, 10⁻⁵, 10⁻¹, 1, 10, 25, 50, 75, ou 90% alors que la variation de luminescence est associée à au moins une propriété de luminescence qui varie de plus de 10⁻¹⁰, 10⁻⁵, 10⁻¹, 1, 10, 25, 50, 75, ou 90%, où ce pourcentage de variation peut être mesuré au cours du temps ou entre au moins deux positions spatiales différentes de la particule. La particule selon l'invention de procédé est un assemblage d'atomes, de molécules, à l'état solide, liquide ou gazeux, préférentiellement à l'état solide.

Dans un mode de réalisation de l'invention de procédé, la particule n'est pas ou ne contient pas de liquide cristallin ou de dendrimère.

Le composé est une substance luminescente.

La substance luminescente est une substance capable d'émettre de la lumière lorsqu'elle est ou a été exposée à un rayonnement lumineux. Elle peut être phosphorescente ou fluorescente. Dans un mode de réalisation de l'invention de procédé, NANOF désigne une nanoparticule d'oxyde de fer ferrimagnétique ou ferromagnétique, aussi parfois appelée de manière équivalente nanoparticule, la nanoparticule ou la nanoparticule d'oxyde de fer.

Une nanoparticule d'oxyde de fer ferrimagnétique ou ferromagnétique selon l'invention de procédé, désignée ici par NANOF, possède au moins l'une des propriétés suivantes: (i), un coeur habituellement cristallisé, où cette cristallinité peut être caractérisée par la présence d'au moins un plan cristallin, préférentiellement au moins cinq plans cristallins, plus préférentiellement au moins dix plans cristallins, et dont la composition est de l'oxyde de fer, préférentiellement au moins en partie constituée de maghémite, de magnétite ou d'un mélange de maghémite et de magnétite, le plus préférentiellement une composition majoritairement à base de magnétite, (ii), un revêtement, aussi parfois appelé membrane, soit d'origine biologique, composé par exemple de lipides et de protéines, soit d'origine non biologique, où ce revêtement peut permettre de favoriser un transfert d'énergie ou de particule entre le composé et la NANOF, de stabiliser la NANOF, de la rendre biocompatible, de favoriser son internalisation cellulaire, de la coupler à différents types de substances dont la substance luminescente, une substance d'intérêt médical, de faible abondance naturelle, (iii), un comportement habituellement ferrimagnétique ou ferromagnétique, notamment aux températures physiologiques, (iv), un seul domaine magnétique appelé monodomaine, (v), un possible arrangement en chaîne empêchant ou limitant l'agrégation et pouvant favoriser l'internalisation cellulaire, (vi), une taille mesurée en présence ou en absence d'un revêtement comprise entre 1 nm et 1 µm, entre 10 nm et 200 nm, entre 20 nm et 150 nm, (vii), la présence possible d'une charge, préférentiellement une charge de surface, où cette charge peut être négative, notamment lorsque les nanoparticules sont recouvertes de lipides négativement chargés, où cette charge peut varier en fonction du pH, de l'existence ou non d'un revêtement, et peut favoriser l'internalisation cellulaire, notamment lorsqu'elle est positive ou négative, (viii), au moins un atome métallique, tel qu'un atome de fer, et au moins un autre atome, tel qu'un atome d'oxygène, dont les moments magnétiques sont préférentiellement antiparallèles, menant à des propriétés qui peuvent être ferrimagnétiques, ou (ix), au moins un atome métallique, tel qu'un atome de fer, et au moins un autre atome, tel qu'un autre atome de fer, dont les moments magnétiques sont préférentiellement parallèles, menant à des propriétés qui peuvent être ferromagnétiques.

Dans un mode de réalisation de l'invention de procédé, la nanoparticule d'oxyde de fer est soit entièrement ferrimagnétique soit entièrement ferromagnétique, mais il peut arriver dans certains cas que l'alignement des moments magnétiques diffère entre la surface et le coeur de la nanoparticule, par exemple lorsqu'on considère une nanoparticule d'oxyde de fer dont le coeur contient du fer et dont la surface est de l'oxyde de fer.

Dans un mode de réalisation de l'invention de procédé, la nanoparticule d'oxyde de fer est ferrimagnétique ou ferromagnétique en dessous de la température de Curie ou de la température de fusion de la nanoparticule ou au-dessus de la température de blocage de la nanoparticule ou à un pH de 7, 6, 5, 4, 8, 9 ou 10, ou lorsque l'énergie d'anisotropie de la nanoparticule est plus élevée que l'énergie thermique de la nanoparticule, pouvant mener à un moment magnétique stable thermiquement de la nanoparticule.

Dans certains modes de réalisation de l'invention, il peut arriver que la nanoparticule perde ses propriétés ferromagnétiques ou ferrimagnétiques, par exemple lorsqu'elle est dissoute *in vivo,* lorsqu'elle est internalisée dans des cellules ou des lysosomes, notamment à des pH qui diffèrent significativement du pH physiologique.

Le comportement ferrimagnétique ou ferromagnétique des NANOF résulte en un moment magnétique stable thermiquement, notamment aux températures physiologiques et en une coercivité, aimantation rémanente, aimantation à saturation, anisotropie magnétocristalline, capacité à produire de la chaleur sous l'application d'un champ magnétique alternatif non nulle(s), préférentiellement élevée(s). La NANOF peut ainsi avoir une coercivité supérieure à 1, 10, 100, 200, 500, 1000, ou 10000 Oe, un rapport entre aimantation rémanente et aimantation à saturation supérieur à 0,01, 0,1, 0,2, ou 0,5, une aimantation à saturation supérieure à 0,1, 1, 10, 20, 50, 60, 80, 90, ou 100 emu par gramme de NANOF, ou un taux d'absorption spécifique (SAR en anglais) supérieur à 0,01, 0,1, 1, 10, 100, 200, 500, ou 1000 Watt par gramme de NANOF. La coercivité, le rapport entre aimantation rémanente et aimantation à saturation, ou l'aimantation à saturation, sont préférentiellement mesurés à une température supérieure à 0,1, 1, 10, 50, 100, 200, 400, ou 800 K. La SAR est préférentiellement mesurée à une concentration en NANOF supérieure à 0,01, 0,1, 1, 5, 10, 15, 20, 50, 100, ou 200 mg par ml, préférentiellement en appliquant un champ magnétique alternatif d'intensité supérieure ou inférieure à 0,1, 1, 2, 5, 10, 20, 50, 100, 1000, ou 10000 mT et/ou de fréquence supérieure ou inférieure à 1, 10, ou 100 Hz, 1, 10, 100, 1000, ou 10000 KHz. Ces propriétés magnétiques sont en général meilleures que celles des nanoparticules d'oxyde de fer superparamagnétiques, appelées SPION, qui sont couramment utilisées pour les applications, notamment médicales. Les SPION sont de plus petites tailles que les NANOF et possèdent un moment magnétique habituellement instable thermiquement aux températures physiologiques. Le comportement ferrimagnétique ou ferromagnétique des NANOF peut aussi se traduire par au moins l'une des propriétés suivantes: (i), une interaction entre le moment magnétique des NANOF et un dipôle, moment magnétique, de la substance luminescente résultant en une atténuation de la luminescence de cette substance luminescente, habituellement plus efficace que pour les SPION, (ii), un couplage entre le moment magnétique des NANOF et un rayonnement, préférentiellement un champ magnétique, plus préférentiellement un champ magnétique alternatif, permettant d'induire une variation de luminescence, préférentiellement plus importante que pour les SPION, (iii), un mouvement dans l'organisme du composé ou de la particule pouvant plus facilement être induit par l'application d'un champ magnétique, préférentiellement un gradient de champ magnétique, plus préférentiellement un gradient de champ magnétique alternatif, ou (iv), une internalisation cellulaire du composé ou de la particule favorisée par l'application d'un champ magnétique, préférentiellement un champ magnétique alternatif, pouvant notamment permettre au composé ou à la particule de mesurer en intracellulaire une variation de l'environnement du composé ou de la particule ou les propriétés d'un rayonnement auquel elle est exposée.

La NANOF est associée au composé ce qui est équivalent à dire que le composé est associé à la NANOF. Dans ce cas, le composé possède au moins l'une des propriétés suivantes. Il peut être : (i), lié à la NANOF, (ii), en interaction avec la NANOF, (iii), intégré à la NANOF, notamment lors de la fabrication de la particule, (iv), contenu dans la NANOF, (v), issu de la NANOF, notamment lorsqu'il se détache de la NANOF lors du fonctionnement de la particule, (vi), incorporé ou adsorbé au coeur cristallisé de la NANOF, (vii), incorporé ou adsorbé au revêtement couvrant la NANOF, (viii), dans l'environnement de la NANOF, (ix), en contact direct avec la NANOF, c'est-à-dire notamment sans qu'il y ait de substance entre le composé et la NANOF, ou (x), en contact indirect avec la NANOF, c'est-à-dire notamment qu'au moins une substance, un polymère, ou un solvant, est positionné entre la NANOF et le composé.

Dans le procédé de l'invention, la nanoparticule n'est pas liée au composé par au moins un acide nucléique ou par au moins un acide aminé.
Dans un mode de réalisation de l'invention de procédé, la substance, polymère, ou solvant, positionné entre la NANOF et le composé, n'est pas synthétisé par un organisme vivant, n'est pas une protéine, un lipide, une enzyme, de l'ADN, un brin d'ADN, de l'ARN, un brin d'ARN, un ou plusieurs acide(s) nucléique(s), un ou plusieurs acide(s) aminé(s), n'est pas une substance biologique active, ou n'est pas de la stréptavidine ou de la biotine, Dans ce cas, la NANOF peut ne pas être liée au composé par au moins une telle substance, polymère ou solvant, positionné entre la NANOF et le composé.

Dans un mode de réalisation de l'invention de procédé, la NANOF n'est pas liée au composé par au moins 1, 10, 10², 10³, 10⁶, 10¹⁰, 10²⁰, ou 10⁴⁰ acide(s) aminé(s), acide(s) nucléique(s), brin(s) d'ADN, brin(s) d'ARN, protéine(s), lipide(s), ou enzyme(s).

Dans un mode de réalisation de l'invention de procédé, la substance, polymère, ou solvant, positionné entre la NANOF et le composé, peut être au moins un atome, tel que du carbone, ou complexe d'atomes, issu d'un organisme vivant, provenant d'une protéine, un lipide, de l'ADN, ou de l'ARN, où cet atome ou complexe d'atomes a préférentiellement été dénaturé par un traitement chimique et/ou thermique.

Dans un mode de réalisation de l'invention de procédé, la substance, polymère, ou solvant, positionné entre la NANOF et le composé, représente moins de 90, 50, 25, 10, 5, 1, ou 0.1% de la masse de la NANOF, où ce pourcentage peut correspondre à la masse de la substance, polymère, ou solvant, divisée par la masse de la NANOF.

Dans un mode de réalisation de l'invention de procédé, la substance, le polymère, ou le solvant, positionné entre la NANOF et le composé, n'est pas dénaturé ou détruit, suite à la perturbation physico-chimique exercée sur la particule, ou n'est pas responsable de la dissociation du composé de la NANOF, suite à la perturbation physico-chimique exercée sur la particule. Cette dénaturation ou destruction peut être associée à une perte ou diminution d'activité de la substance, polymère, ou solvant, telle qu'une perte ou diminution d'activité d'une protéine, d'un lipide, d'une enzyme, une transformation d'ADN double brin en ADN simple brin, ou une perte d'au moins un acide aminé ou d'au moins un acide nucléique par un complexe d'acides aminés ou d'acides nucléiques.

Dans un mode de réalisation, la NANOF est responsable de la dissociation du composé de la NANOF, suite à la perturbation physico-chimique exercée sur la particule.

Dans un mode de réalisation de l'invention de procédé, le composé est en contact direct avec la NANOF lorsque la distance entre le composé et la NANOF est inférieure à 10 µm, 1 µm, 100 nm, 10 nm, ou 1 nm.

L'environnement de la NANOF peut être un milieu liquide, solide ou gazeux ou au moins 1, 10, 10², 10³, 10⁶, 10¹⁰, ou 10⁴⁰ substance(s), préférentiellement une ou des substance(s) différente(s) du composé, qui entoure(nt) ou inclus(en)t la particule sur une distance mesurée à partir du centre ou de la surface extérieure de la particule de préférence inférieure à 1 m, 1 dm, 1 cm, 1 mm, 1 µm, 100 nm, ou 10 nm.

L'environnement de la NANOF peut être un milieu liquide, solide ou gazeux ou au moins 1, 10, 10², 10³, 10⁶, 10¹⁰, ou 10⁴⁰ substance(s), préférentiellement une ou des substance(s) différente(s) du composé, qui entoure(nt) ou inclus(en)t la particule sur une distance mesurée à partir du centre ou de la surface extérieure de la particule de préférence supérieure à 1 m, 1 dm, 1 cm, 1 mm, 1 µm, 100 nm, ou 10 nm.

L'environnement de la NANOF peut être un milieu liquide, solide ou gazeux ou au moins 1, 10, 10², 10³, 10⁶, 10¹⁰, ou 10⁴⁰ substance(s), préférentiellement une ou des substance(s) différente(s) du composé, qui entoure ou entourent ou inclus ou incluent la particule sur une distance mesurée à partir du centre ou de la surface extérieure de la particule de préférence inférieure ou supérieure au diamètre de la terre, ou à 1000 km, ou à 1 km, ou à 1 m, ou à 1 dm, ou à 1 cm, ou à 1 mm, ou à 1 µm, ou à 100 nm, ou à 10 nm.

Selon l'invention de procédé, le composé est dit se dissocier de la NANOF lorsqu'il se sépare de la NANOF pour se retrouver dans l'environnement de la NANOF.

Dans un mode de réalisation de l'invention de procédé, la particule est inactivée ou est dans un état inactivé lorsqu'aucune perturbation physico-chimique n'est exercée sur elle.

Dans un autre mode de réalisation, la particule est activée ou est dans un état activé lorsqu'une perturbation physico-chimique est exercée sur elle.

La sonde selon l'invention de procédé peut contenir la particule dans un état activé et/ou inactivé.

Une NANOF et/ou un composé et/ou un magnétosome, modifié ou non, peut ou peuvent, selon l'invention de procédé, être dans un état activé ou être dans état inactivé.

Une perturbation physico-chimique peut être exercée à l'aide d'un appareil fabriqué par l'homme, tel qu'un IRM, ou de manière naturelle par l'environnement naturel de la particule, tel qu'un environnement radioactif.

La dissociation du composé de la NANOF peut être caractérisée par une constante de dissociation, K_{D}, pouvant être égale au rapport entre la concentration du composé lié à la NANOF, mesurée lorsque la particule est activée, divisée par cette même concentration, mesurée lorsque la particule est inactivée. De préférence, la constante K_{D} selon l'invention de procédé peut être inférieure à 1, 0,9, 0,8, 0,7, 0,6, 0,5, 0,4, 0,3, 0,2, 0,1, 0,05, 0,02, 0,1, 0,05, 0,01, 10⁻³, 10⁻⁴, 10⁻⁵, 10⁻⁶, 10⁻⁷, 10⁻⁸, ou 10⁻⁹. Suivant cette définition, plus K_{D} est faible, plus la dissociation du composé de la NANOF est importante.

Une mesure de la force de liaison ou d'interaction entre le composé et la NANOF peut être donnée par la valeur de la constante de dissociation, K_{D}. Plus K_{D} a une valeur élevée, plus la liaison entre le composé et la NANOF est forte.

Le composé est dit se modifier chimiquement lorsque sa composition et/ou sa structure change(nt), induisant une modification de certaines de ses propriétés, notamment de luminescence et/ou pharmacologiques.

Dans l'invention de procédé, la dissociation du composé n'entraine(nt) pas sa destruction ou sa dénaturation ou préserve le composé. Dans ce cas, la dissociation du composé n'entraine(nt) préférentiellement pas sa dénaturation ou destruction totale ou partielle, ou la perte d'au moins 1, 10, 10², 10⁵, 10¹⁰, ou 10²⁰ atomes, ou la perte d'au moins 0,1, 1, 5, 10, 25, 50, ou 90% de son poids, ou la perte d'au moins l'une de ses propriétés telle que la perte d'une propriété de luminescence ou d'une propriété pharmacologique, ou la diminution de plus de 1, 5, 10, 25, 50, 90% de son intensité de luminescence, ou la perte de son activité thérapeutique ou de diagnostic

La perturbation physico-chimique exercée sur la NANOF d'oxyde de fer peut induire un changement d'état de la NANOF, préférentiellement de manière sélective sans induire un tel changement d'état chez le composé. Cela peut être le cas lorsque la NANOF est exposée à une perturbation physico-chimique qui induit un changement d'état tel qu'un mouvement, ou une augmentation de température de la NANOF. La perturbation physico-chimique peut induire un plus grand changement d'état, tel qu'une plus grande variation de température, un mouvement plus important ou une modification plus importante d'une propriété chez la NANOF que chez

le composé. Afin de mettre en évidence cette différence de changement d'état, la perturbation physico-chimique peut être exercée sur la NANOF seule et sur le composé seul et la modification d'une des propriétés de la NANOF seule peut être mesurée et être plus importante que la modification de cette même propriété chez le composé seul, suite à l'application de cette perturbation.

La perturbation physico-chimique est de préférence une perturbation qui ne modifie pas de manière irréversible les propriétés du composé ou de la NANOF, préférentiellement les propriétés de luminescence ou de substance thérapeutique du composé. Une telle perturbation, menant à la dénaturation du composé ou de la NANOF, consistant en une augmentation de température, au-delà de la température de fusion ou de dénaturation du composé ou de la NANOF, est par exemple préférentiellement évitée. On préfère que la perturbation physico-chimique soit suffisamment modérée pour que le composé préserve au moins en partie ses propriétés, préférentiellement de luminescence ou de substance thérapeutique, suite à l'application de cette perturbation.

Une perturbation physico-chimique est de préférence une perturbation suffisamment modérée, telle qu'une augmentation modérée de température, préférentiellement inférieure à 300, 200, 100, 50, 20, 10, ou 1 °C, de la particule qui peut résulter en une perte de masse de la particule de moins de 90, 50, 25, 10, 5, 1, ou 0,1 %.

Un rayonnement peut être un rayonnement ondulatoire ou particulaire, sonore, ionisant, un rayonnement de rayons X, électromagnétique, un rayonnement du à un champ électrique, magnétique ou électromagnétique, ou un rayonnement causé par des neutrons, protons, électrons, positron, particules alpha, béta, gamma, des neutrinos ou des muons.

L'environnement de la particule peut être un milieu liquide, solide ou gazeux ou au moins 1, 10, 10², 10³, 10⁴, 10⁵, 10⁶, 10⁹, 10²⁰, ou 10⁴⁰ substance(s) qui entoure(nt) ou inclu(en)t la particule sur une distance mesurée à partir du centre ou de la surface externe de la particule de préférence inférieure à 10 000 km, 1 000 km, 100 km, 10 km, 1 km, 1 m, 1 dm, 1 cm, 1 mm, 1 µm, 100 nm, ou 10 nm.

L'environnement de la particule selon l'invention de procédé peut être un milieu liquide, solide ou gazeux ou au moins 1, 10, 10², 10³, 10⁴, 10⁵, 10⁶, 10⁹, 10²⁰, ou 10⁴⁰ substance(s) qui entoure(nt) ou inclus(en)t la particule sur une distance mesurée à partir du centre ou de la surface externe de la particule de préférence supérieure à 10 000 km, 1 000 km, 100 km, 10 km, 1 km, 1 m, 1 dm, 1 cm, 1 mm, 1 µm, 100 nm, ou 10 nm.

L'environnement de la particule selon l'invention de procédé peut être un milieu liquide, solide ou gazeux ou au moins 1, 10, 10², 10³, 10⁴, 10⁵, 10⁶, 10⁹, 10²⁰, ou 10⁴⁰ substance(s) qui entoure ou entourent ou inclus ou incluent la particule sur une distance mesurée à partir du centre ou de la surface externe de la particule de préférence supérieure ou inférieure au diamètre de la terre, ou à 10 000 km, ou à 1 000 km, ou à 100 km, ou à 10 km, ou à 1 km, ou à 1 m, ou à 1 dm, ou à 1 cm, ou à 1 mm, ou à 1 µm, ou à 100 nm, ou à 10 nm.

L'environnement du composé selon l'invention de procédé peut être un milieu liquide, solide ou gazeux ou au moins 1, 10, 10², 10³, 10⁴, 10⁵, 10⁶, 10⁹, 10²⁰, ou 10⁴⁰ substance(s) qui entoure ou entourent ou inclus ou incluent le composé sur une distance mesurée à partir du centre ou de la surface externe du composé de préférence supérieure ou inférieure au diamètre de la terre, ou à 10 000 km, ou à 1 000 km, ou à 100 km, ou à 10 km, ou à 1 km, ou à 1 m, ou à 1 dm, ou à 1 cm, ou à 1 mm, ou à 1 µm, ou à 100 nm, ou à 10 nm.

L'environnement de la particule selon l'invention de procédé peut inclure la particule, le composé et/ou la NANOF.

Une substance de l'environnement de la particule, du composé ou de la NANOF selon l'invention de procédé peut être un atome, une molécule, un polymère, une substance chimique ou biologique, préférentiellement une substance différente du composé ou de la NANOF, de l'ADN, de l'ARN, une protéine, un lipide, une enzyme, ou un acide nucléique ou aminé contenu dans cet environnement.

L'environnement de la particule, du composé ou de la NANOF selon l'invention de procédé peut être un environnement biologique, c'est-à-dire un environnement comprenant au moins une substance biologique telle qu'une cellule, une organelle, de l'ADN, ARN, une protéine, un lipide, un acide aminé, ou un acide nucléique.

La variation de l'environnement de la particule, de la NANOF ou du composé selon l'invention de procédé peut être une variation de pH, préférentiellement inférieure ou supérieure à 14, 13, 12, 11, 10, 9, 8, 7, 6, 5, 4, 3, 2, 1, ou 0,1 unités de pH, une variation de température, préférentiellement inférieure ou supérieure à 500, 400, 300, 200, 100, 50, 25, 10, 5, 1, ou 0,1 °C.

Dans la présente description, on distingue notamment le cas où la particule est inactivée et ne subit pas de perturbation physico-chimique de celui où elle est activée et subit une telle perturbation.

Les conditions de fonctionnement de la particule selon l'invention de procédé correspondent aux conditions dans lesquelles la particule passe d'un état inactivé à un état activé ou *vice versa* d'un état activé à un état inactivé.

Pour certains modes de fonctionnement de la particule selon l'invention de procédé, le composé est en plus forte interaction avec la NANOF lorsque la particule est inactivée que lorsque la particule est activée.

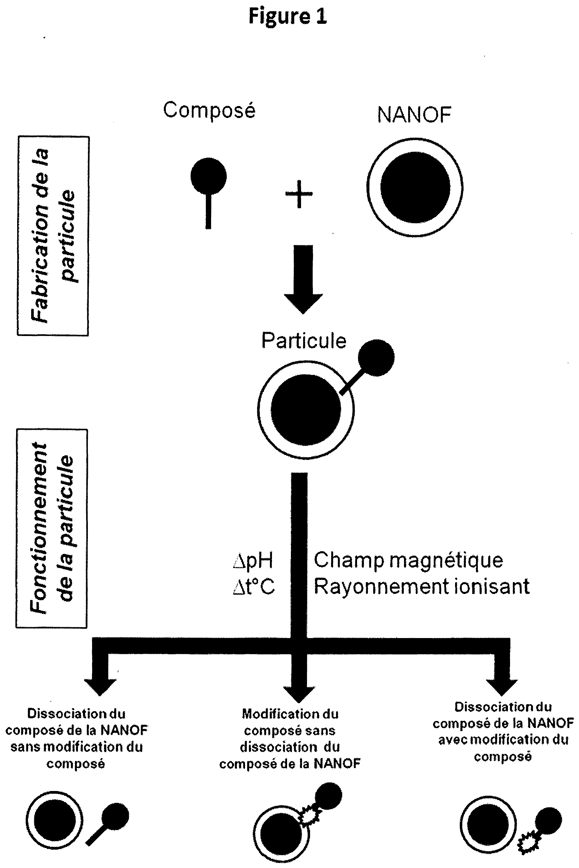

Un schéma est présenté Figure 1 qui illustre le fonctionnement de la particule décrite en faisant intervenir soit une dissociation du composé de la NANOF (procédé revendiqué) soit une modification chimique du composé (comparatif).

Dans un mode de réalisation de l'invention de procédé, la nanoparticule d'oxyde de fer ferrimagnétique ou ferromagnétique (NANOF) est synthétisée par une bactérie magnétotactique et ne comprend pas de matière carbonée provenant de la bactérie.

Dans un mode de réalisation de l'invention de procédé, la NANOF est synthétisée par un organisme vivant, préférentiellement une bactérie, plus préférentiellement une bactérie magnétotactique.

Suivant un mode de réalisation de l'invention de procédé, les NANOF sont des nanoparticules synthétisées par des bactéries magnétotactiques, notamment sélectionnées dans le groupe constitué de *Magnetospirillum magneticum* AMB-1, les *coccus* MC-1, les *vibrios* MV-1, MV-2 et MV-4, les *Magnetospirillum magneticum* MS-1, les *Magnetospirillum gryphiswaldense* MSR-1, des *spirillum,* les *Magnetospirillum magneticum* MGT-1 et les *Desulfovibrio magneticus* RS-1.

Une NANOF synthétisée par une bactérie magnétotactique est un magnétosome, notamment tel que défini par D. A. Bazylinski et al, Nature Reviews Microbiology, V. 2, P. 217 (2004). Une NANOF, selon l'invention de procédé, peut être synthétisée par voie chimique ou par un autre organisme vivant qu'une bactérie magnétotactique et avoir au moins une propriété identique ou similaire à celle d'un magnétosome, modifié ou non.

Un magnétosome, selon l'invention de procédé, peut avoir au moins une propriété identique ou similaire à celle d'une NANOF.

Dans un mode de réalisation de l'invention de procédé, les magnétosomes sont sous forme de chaîne contenant de 2 à 1000 magnétosomes, de 2 à 500 magnétosomes, de 2 à 250 magnétosomes, de 2 à 100 magnétosomes, de 2 à 75 magnétosomes, typiquement de 4 à 20 magnétosomes, préférentiellement maintenus arrangés en chaînes par du matériau, d'origine biologique (lipides et protéines par exemple) ou non biologique.

Les magnétosomes appartenant à ces chaînes possèdent habituellement des directions cristallographiques, des axes de facile aimantation, ou des lignes de champ, qui sont alignés dans la direction de l'élongation des chaînes. Cet alignement permet entre autre de stabiliser le moment magnétique des magnétosomes. Par conséquent, les chaînes de magnétosomes possèdent une anisotropie magnétique importante. Lorsque plusieurs chaînes de magnétosomes contenant au moins X magnétosomes interagissent, il en résulte la formation d'une plus longue chaîne de magnétosomes contenant au moins X+1 magnétosomes.

La longueur d'une chaîne de magnétosomes selon l'invention de procédé peut être inférieure ou supérieure à 1 m, 1 dm, 1 cm, 1 mm, 100 µm, 10 µm, 1 µm, 600 nm, 300 nm. Du fait de leur arrangement en chaînes, les magnétosomes ne s'agrègent pas et possèdent un moment magnétique stable qui peut se coupler au champ magnétique, favorisant ainsi la variation de luminescence de ladite sonde. L'arrangement en chaînes permet également de favoriser l'internalisation cellulaire des magnétosomes, elle-même renforcée par l'application d'un champ magnétique alternatif.

Dans un mode de réalisation de l'invention de procédé, afin de déterminer si les NANOF ou magnétosomes sont sous forme de chaînes ou non, quelques microlitres d'une suspension de NANOF sont déposés sur un support, habituellement une grille de carbone, et les NANOF sont analysées au microscope électronique. Les NANOF sont considérées comme arrangées en chaîne lorsqu'elles possèdent des axes ou directions cristallographiques, notamment au moins 1, 2, 5, 10, 20, 30, 50, alignés dans la direction d'élongation d'une chaîne. Un tel alignement peut signifier que l'angle entre la direction cristallographique d'une NANOF et la direction d'élongation de la chaîne est inférieur à 90, 80, 50, 40, 20, 10, ou 5°. Les NANOF ne possédant pas cette propriété ne sont pas arrangées en chaînes.

Dans un mode de réalisation de l'invention de procédé, les bactéries magnétotactiques peuvent être cultivées dans un milieu, tel que celui décrit dans la demande de brevet WO2011/061259 incorporée par référence.

Dans un mode de réalisation de l'invention de procédé, le milieu de culture des bactéries magnétotactiques contient au moins une source de fer, telle qu'une solution de quinate de fer, de citrate de fer, de chlorure de fer, de malate de fer, ou de sulfate ferreux, notamment à des concentrations comprises entre 0,02 µM et 2 M, 0,02 µM et 200 mM, 0,2 µM et 20 mM, 2 µM et 2 mM, ou entre 20 µM et 200 µM et un ou plusieurs autres additifs habituellement ajouté(s) à des concentrations comprises entre 0,01 µM et 1 M, 0,01 µM et 100 mM, 0,01 µM et 10 mM, 0,01 µM et 1 mM, 0,1 µM et 100 µM, ou entre 1 µM et 10 µM. Ces additifs sont préférentiellement choisis parmi les métaux de transition, les agents chélateurs, les substances luminescentes, les substances/isotopes de faible fréquence/abondance naturelle, les substances ayant les propriétés de plusieurs des substances précitées. Des bactéries ou ces additifs peuvent être introduits dans le milieu de culture lors d'au moins une des différentes phases de croissance des bactéries magnétotactiques, telle que la phase de latence, la phase d'accélération, la phase exponentielle, la phase de décélération ou la phase stationnaire et/ou les bactéries peuvent être cultivées en présence d'une faible concentration d'oxygène, inférieure à 1000 bar, 100 bar, 10 bar, 1 bar, 200 mbar, ou 20 mbar et/ou le pH ou un autre paramètre du milieu de croissance des bactéries magnétotactiques est maintenu à une valeur constante ou quasi constante pendant la croissance des bactéries magnétotactiques, par exemple par ajout d'une solution acide ou basique contenant préférentiellement une certaine concentration de ces additifs. Par ailleurs, ces additifs et conditions de croissance des bactéries sont préférentiellement choisis parmi ceux qui permettent d'éviter la toxicité, qui confèrent de bonnes propriétés magnétiques et/ou de luminescence à ladite sonde, qui permettent aux additifs de s'incorporer dans les bactéries magnétotactiques et/ou dans les magnétosomes, qui produisent une augmentation de taille et/ou de longueur des chaînes des magnétosomes, qui stimulent la croissance des bactéries magnétotactiques, qui permettent d' atteindre un rendement de production des magnétosomes suffisamment élevé, notamment supérieur à 0,1 mg, 1 mg, 10 mg, 100 mg, 1g, 10 g de magnétosomes par litre de culture, qui permettent d'obtenir une distribution en tailles des magnétosomes étroite, notamment de moins de 60 nm, 30 nm, 15 nm, 10 nm, 5 nm, ou 1 nm.

Dans un mode de réalisation de l'invention de procédé, l'association aux magnétosomes de la substance luminescente peut être ou peut ne pas être obtenue par manipulation génétique des bactéries magnétotactiques.

Dans l'invention de procédé, la substance luminescente n'est pas une protéine fluorescente, telle qu'une protéine fluorescente verte ou jaune, une enzyme fluorescente, un acide aminé fluorescent, un acide nucléique fluorescent, de l'ADN ou brin d'ADN fluorescent, ou de l'ARN ou brin d'ARN fluorescent.

Dans un mode de réalisation de l'invention de procédé, la NANOF est un magnétosome modifié.

Un magnétosome modifié est préférentiellement un magnétosome ne comprenant pas, essentiellement pas, ou en faible quantité, de matière organique ou carbonée, constitué notamment de lipides, protéines, ADN, ARN, ou enzymes, pouvant notamment entourer le coeur minéral des magnétosomes.

Dans un mode de réalisation de l'invention de procédé, un minéral ne contient pas de carbone ou moins de 75%, 50%, 25%, 10%, 5%, 2%, 1%, ou 0,5% de carbone.

Dans un mode de réalisation de l'invention de procédé, un magnétosome modifié selon l'invention de procédé comprend moins de 100%, 90%, 80% , 70%, 60%, 50%, 40%, 30%, 20%, 10%, 5%, 1%, ou 0,1% de matière carbonée, où ce pourcentage de matière carbonée peut être défini comme étant le rapport entre le nombre d'atomes de carbones dans un magnétosome modifié et le nombre total d'atomes contenu dans un magnétosome modifié.

Un magnétosome modifié est préférentiellement un nanocristal constitué majoritairement d'un coeur minéral et possiblement d'un revêtement qui ne provient pas de la bactérie magnétotactique.Un magnétosome modifié peu posséder au moins une propriété en commun avec un magnétosome non modifié, tel qu'un arrangement en chaîne.

Une préparation de magnétosome modifié selon l'invention de procédé est préférentiellement apyrogène, et ne contient pas, essentiellement pas, ou peu d'endotoxines ou de lipopolysaccharides.

Dans un mode de réalisation de l'invention de procédé, une préparation de magnétosome modifié comprend moins de 10⁶, 10⁵, 10⁴, 10³, 10², 10, 1, 10⁻¹, 10⁻², 10⁻³, 10⁻⁴, ou 10⁻⁵ UE (unités d'endotoxines) par mL par mg, par mg, ou par cm³ de magnétosome modifié ou de préparation de magnétosome modifié.

Dans un mode de réalisation de l'invention de procédé, la plus grande dimension de la particule est de préférence comprise entre 1 nm et 10 µm ou 10 nm et 1 µm.

Dans un mode de réalisation de l'invention de procédé, la plus grande dimension de la particule est de préférence inférieure à 100 µm, 50 µm, 10 µm, 1 µm, 500 nm, 250 nm, 100 nm ou 50 nm.

Dans un mode de réalisation, la particule selon l'invention de procédé comprend en outre une substance sélectionnée dans le groupe constitué d'un isotope de faible fréquence ou abondance naturelle, d'un métal de transition, et d'une substance d'intérêt médical.

Avantageusement, un isotope de faible fréquence ou abondance naturelle peut être associé à la particule pour faciliter la détection de la particule dans un milieu contenant peu ou pas de cet isotope (par exemple le fer 54, 57 ou 59 qui sont peu présents à l'état naturel). Avantageusement également, un métal de transition peut être associé à la particule afin d'en améliorer les propriétés magnétiques, permettant notamment de faciliter la détection de sa variation de luminescence.

Avantageusement également, une substance d'intérêt médical tel qu'un agent ciblant, une substance utilisée lors d'une opération médicale, d'une thérapie, d'un diagnostic, d'une séance de chirurgie, radiothérapie, hyperthermie, IRM, chimiothérapie, peut être associée à la particule, afin notamment de faciliter ou d'améliorer son utilisation ou efficacité médicale. Dans un mode de réalisation de l'invention de procédé, la particule selon l'invention de procédé peut être encapsulée dans un compartiment, préférentiellement une vésicule, le plus préférentiellement une vésicule lipidique telle qu'un liposome, une vésicule géante ou un nanogel, notamment afin d'améliorer l'efficacité ou de diminuer la toxicité de la particule. Dans un mode de réalisation de l'invention de procédé, la nanoparticule d'oxyde de fer ferrimagnétique ou ferromagnétique (NANOF) est synthétisée par voie chimique.

Dans l'invention de procédé, le composé est associé à la NANOF par des liaisons faibles. Cette situation est rencontrée lorsque le fonctionnement de la particule selon l'invention de procédé fait intervenir une dissociation du composé de la NANOF.

Les liaisons faibles sont préférentiellement des liaisons permettant la dissociation du composé de la particule sous l'effet d'une perturbation physico-chimique où cette perturbation est préférentiellement telle qu'elle ne modifie pas de manière significative ou qu'elle modifie de manière non significative l'une au moins des propriétés de la NANOF telle que sa taille, sa distribution en taille, sa propriété ferrimagnétique ou ferromagnétique, sa composition, ou sa structure cristallographique.

Dans un mode de réalisation de l'invention de procédé, une modification significative d'une propriété de la NANOF peut être définie comme étant: (i), une augmentation ou une diminution de la taille des NANOF de plus de 100 nm, 1 µm, ou 10 µm, (ii), une augmentation ou une diminution de la distribution en taille des NANOF de plus de 100 nm, 1 µm, ou 10 µm, (iii), un changement de propriété magnétique dû par exemple au passage de la NANOF de nanoparticule avec un seul domaine magnétique à une nanoparticule avec plusieurs domaines magnétiques, pouvant notamment entraîner la perte de propriété ferrimagnétique ou ferromagnétique de la NANOF, (iv), un changement de composition tel que le passage d'une nanoparticule composée d'oxyde de fer à une nanoparticule ne contenant plus que du fer ou de l'oxygène, ou (v), une modification de la structure cristallographique telle que le passage de la nanoparticule d'un état cristallisé, pouvant être caractérisé par la présence d'au moins deux plans cristallins dans la nanoparticule à un état amorphe, pouvant être caractérisé par l'absence de plans cristallins dans la nanoparticule.

Dans un mode de réalisation de l'invention de procédé, une modification non significative d'une propriété de la NANOF peut être définie comme étant: (i), une augmentation ou une diminution de la taille des NANOF de moins de 100 nm, 1 µm, ou 10 µm, (ii), une augmentation ou une diminution de la distribution en taille des NANOF de moins de 100 nm, 1 µm, ou 10 µm, (iii), une absence de changement de propriété magnétique dû par exemple au passage de la NANOF de nanoparticule avec un seul domaine magnétique à une nanoparticule avec plusieurs domaines magnétiques, pouvant notamment entraîner la perte de propriété ferrimagnétique ou ferromagnétique de la NANOF, (iv), une absence de changement de composition tel que le passage d'une nanoparticule composée d'oxyde de fer à une nanoparticule ne contenant plus que du fer ou de l'oxygène, ou (v), une absence de modification de la structure cristallographique telle que le passage de la nanoparticule d'un état cristallisé, pouvant être caractérisé par la présence d'au moins deux plans cristallins dans la nanoparticule à un état amorphe, pouvant être caractérisé par l'absence de plans cristallins dans la nanoparticule.

De préférence, les liaisons faibles selon l'invention de procédé sont des liaisons hydrogènes, des liaisons qui sont dues à des forces de Van der Waals, de London, de Debye, des forces intermoléculaires ou inter-nanoparticulaires, ou des interactions hydrophobes ou hydrophiles ou de nature électrostatique.

Dans un mode de réalisation de l'invention de procédé, les liaisons faibles peuvent être associées à l'adsorption du composé sur la NANOF.

Dans un mode de réalisation de l'invention de procédé, les liaisons faibles peuvent être caractérisées par une constante de dissociation, K_{D}, qui est de préférence inférieure à 1, 0,9, 0,8, 0,7, 0,6, 0,5, 0,4, 0,3, 0,2, 0,1, 0,05, 0,02, 0,1, 0,05, 0,01, 10⁻³, 10⁻⁴, 10⁻⁵, 10⁻⁶, 10⁻⁷, 10⁻⁸, ou 10⁻⁹.

Dans un mode de réalisation de l'invention de procédé, la liaison entre le composé et la NANOF est plus forte lorsque la particule est inactivée que lorsqu'elle est activée. Cette propriété peut notamment être observée lorsque le fonctionnement de la particule fait intervenir une dissociation du composé de la NANOF.

Dans l'invention de procédé, le composé n'est pas lié à la NANOF par des liaisons fortes, telles que des liaisons covalentes, ioniques, ou par des liaisons qui empêchent une dissociation du composé de la NANOF. Cette propriété est préférentiellement observée lorsque le fonctionnement de la particule fait intervenir une dissociation du composé de la NANOF.

Dans l'invention de procédé, la particule contient un composé qui est une substance luminescente.

La substance luminescente selon l'invention de procédé possède au moins une propriété de luminescence.

Une propriété de luminescence est définie ici comme étant une intensité, une longueur d'onde de luminescence, une intensité, une longueur d'onde d'absorption, un temps de vie de luminescence, ou une anisotropie de luminescence, qui est préférentiellement mesurable. La propriété de luminescence peut être attribuée à la substance luminescente ou à la particule.

Dans l'invention de procédé, la substance luminescente possède au moins une propriété de luminescence qui varie, préférentiellement dans les conditions de fonctionnement de la particule.

Une variation de luminescence est définie ici comme étant une variation d'au moins une propriété de luminescence. Elle peut être attribuée à la substance luminescente ou à la particule et peut être une augmentation ou une diminution, préférentiellement une augmentation, d'au moins une propriété de luminescence de la particule ou de la substance luminescente.

Une propriété de luminescence peut varier au cours du temps, en fonction de la position spatiale de la particule ou de la NANOF, lors d'une variation de l'environnement de la particule ou de la NANOF, suite à l'application du rayonnement, de la dissociation de la substance luminescente de la NANOF.

Dans un mode de réalisation de l'invention de procédé, la variation de luminescence est une augmentation continue de la luminescence. Elle se produit préférentiellement au cours du temps, sur un intervalle de temps de préférence supérieur à 10⁻¹⁰, 10⁻⁹, 10⁻⁸, 10⁻⁷, 10⁻⁶, 10⁻⁵, 10⁻⁴, 10⁻³, 10⁻², 10⁻¹, 1, 2, 5, ou 10 secondes, 1, 2, 5, 10, 30, ou 45 minutes, 1, 2, 5, 10, ou 17 heures, 1, 2, 5, 10, 15, ou 30 jours, 1, 2, 5, ou 10 mois, 1, 2, 5, 10, 25, 50, ou 100 ans. Elle n'est préférentiellement pas une succession d'au moins 1, 10, 10², 10³, 10⁶, 10⁹, 10²⁰, ou 10⁴⁰ augmentation(s) et diminution(s) de luminescence pouvant notamment se produire lors d'interactions aléatoires de la substance luminescente.

Dans un mode de réalisation de l'invention de procédé, la variation de luminescence (ΔL) au cours du temps est caractérisée par une pente à l'origine, ΔL/δt, où δt représente une durée ayant pour origine le début de la variation de luminescence, qui est préférentiellement déterminée pendant les premières secondes ou minutes suivant la perturbation physico-chimique exercée sur la particule.

Une propriété de luminescence ou sa variation peut être déterminée autour, à la surface ou dans la particule ou NANOF sur une distance mesurée à partir du centre de la particule ou NANOF de préférence inférieure à 1 m, 1 dm, 1 cm, 1 mm, 1 µm, 100 nm, ou 10 nm.

Dans un mode de réalisation de l'invention de procédé, la variation de luminescence est déterminée sur une zone permettant de détecter la variation de luminescence, c'est à dire une zone contenant un nombre suffisant de particules, de préférence supérieur à 1, 10, 100, 10³, 10⁴, 10⁵, 10⁶, 10⁷, 10⁸, 10⁹, 10¹⁰, ou 10¹¹ particules ou particules par unité de volume, notamment particules par m³, cm³, mm³, ou µm³.

Dans un mode de réalisation de l'invention de procédé, la substance luminescente possède au moins une propriété de luminescence qui est mesurable au cours du temps et/ou en fonction de la position de la particule ou de la NANOF, ou subit une variation d'au moins l'une de ses propriétés qui est mesurable au cours du temps et/ou en fonction de la position de la particule ou de la NANOF.

Dans un autre mode de réalisation de l'invention de procédé, certains paramètres comme le temps de mesure, les taille et masse des substances luminescentes et des NANOF, le type de liaison entre substance luminescente et NANOF, ou les propriétés de l'environnement de la particule telle que sa viscosité, sont optimisés pour permettre la mesure d'une variation de luminescence de la substance luminescente.

Dans un mode de réalisation de l'invention de procédé, la substance luminescente possède au moins l'une des propriétés suivantes: (i), une petite taille ou une petite masse, préférentiellement inférieure(s) à un dixième, un centième, ou un millième de celle(s) des NANOF, pouvant notamment permettre de favoriser son décrochage des NANOF et/ou sa diffusion dans l'environnement de la particule, (ii), une charge négative, préférentiellement inférieure à -1, -10, -20, ou -50 mV, une charge positive, préférentiellement supérieure à 1, 10, 20, ou 50 mV, une charge neutre, ou une charge opposée à celle des NANOF pouvant notamment favoriser la liaison entre la substance luminescente et la NANOF, (iii), une liaison avec les NANOF permettant sa dissociation des NANOF dans les conditions de fonctionnement de la particule, (iv), une cristallinité révélée par la présence d'au moins deux plans cristallins, ou (v), une absorption non nulle à des longueurs d'onde où le milieu biologique absorbe peu, ou une absorption non nulle entre 20 nm et 2 µm ou entre 600 et 1200 nm.

Dans un mode de réalisation de l'invention de procédé, la substance luminescente préférée est celle qui permet d'atteindre la plus grande atténuation de l'intensité de luminescence et/ou le plus petit temps de vie de luminescence lorsque la particule est inactivée.

Dans un mode de réalisation de l'invention de procédé, la substance luminescence préférée est celle qui permet le transfert d'énergie ou de particule le plus important avec la NANOF lorsque la particule est inactivée ou activée.

Dans un mode de réalisation de l'invention de procédé, la substance luminescente préférée est celle qui permet d'induire une variation de luminescence qui est : (i), la plus importante possible et/ou mesurable lorsque la particule passe d'un état inactivé à un état activée, ou (ii), mesurable lorsque la particule est exposée à un rayonnement de faible intensité, de faible énergie et/ou à une variation de l' environnement de la particule de faible amplitude, telle qu'une variation de température de moins de 10⁶, 10⁵, 10⁴, 10³, 10², 10, 5, 1, 0,5, ou 0,1 °C ou une variation de pH de moins de 100, 50, 20, 14, 10, 8, 6, 4, 2, 1, 0,5, 0,1, ou 0,01 unités de pH.

Dans un mode de réalisation de l'invention de procédé, au moins une propriété de luminescence de la substance luminescente associée à la nanoparticule d'oxyde de fer diffère de celle de la substance luminescente seule. Cette différence est plus facilement observable et mesurable lorsque la particule est inactivée ou activée, préférentiellement inactivée, que lorsque la particule passe de l'état inactivé à l'état activé ou de l'état activé à l'état inactivé.

Dans un mode de réalisation de l'invention de procédé, l'intensité de luminescence de la substance luminescente associée à la nanoparticule d'oxyde de fer est atténuée par la nanoparticule d'oxyde de fer par rapport à l'intensité de luminescence de la substance luminescente seule. Cette atténuation est préférentiellement observée et mesurable lorsque la particule est inactivée.

Dans un mode de réalisation de l'invention de procédé, l'atténuation de l'intensité de luminescence de la substance luminescente est déterminée en mesurant le rapport I_{L}/I_{NL} entre l'intensité de luminescence de la substance luminescente liée à la NANOF, I_{L}, et l'intensité de luminescence de la substance luminescente seule, non liée à la NANOF, I_{NL}. Ce rapport, notamment I_{L}, est préférentiellement mesuré lorsque la particule est inactivée.

Dans un mode de réalisation de l'invention de procédé, I_{L} et I_{NL} sont mesurées dans les mêmes conditions, notamment en ce qui concerne les paramètres d'excitation et de détection de la luminescence, ainsi que la concentration de la substance luminescente, utilisés lors des mesures. Dans un mode de réalisation de l'invention de procédé, l'intensité de luminescence de la substance luminescente liée à la NANOF est atténuée lorsque I_{L}/I_{NL} est inférieur à 1, préférentiellement inférieur à 0,9, 0,8, 0,7, 0,6, 0,5, 0,4, 0,3, 0,2, 0,1, 0,05, 0,02, 0,01, 0,005, 0,001, 10⁻⁴, 10⁻⁵, 10⁻⁶, 10⁻⁷, 10⁻⁸, ou 10⁻⁹.

Dans un mode de réalisation de l'invention de procédé, le temps de vie de luminescence de la substance luminescente associée à la nanoparticule d'oxyde de fer est modifié, atténué, ou augmenté, préférentiellement augmenté, par la nanoparticule d'oxyde de fer par rapport au temps de vie de luminescence de la substance luminescente seule. Dans le cas où le temps de vie de luminescence est augmenté par la nanoparticule d'oxyde de fer, il s'agit d'une situation inhabituelle et différente de celle habituellement rencontrée dans le cas d'un transfert de Förster, devant mener à une diminution du temps de vie.

Dans un mode de réalisation de l'invention de procédé, la variation du temps de vie de luminescence de la substance luminescente est déterminée en mesurant le rapport T_{A}/T_{L} entre le temps de vie de luminescence de la substance luminescente liée à la NANOF, T_{A}, et le temps de vie de luminescence de la substance luminescente seule, non liée à la NANOF, T_{L}.

Dans un mode de réalisation de l'invention de procédé, T_{A} et T_{L} sont mesurés dans les mêmes conditions, notamment en ce qui concerne les paramètres d'excitation et de détection de la luminescence ainsi que la concentration de la substance luminescente utilisée lors des mesures. Dans un mode de réalisation de l'invention de procédé, le temps de vie de luminescente de la substance luminescente liée à la NANOF est diminué, notamment par la présence de la NANOF, lorsque T_{A}/T_{L} est inférieur à 1, préférentiellement inférieur à 0,9, 0,8, 0,7, 0,6, 0,5, 0,4, 0,3, 0,2, 0,1, 0,05, 0,02, 0,01, 0,005, 0,001, 10⁻⁴, 10⁻⁵, 10⁻⁶, 10⁻⁷, 10⁻⁸, ou 10⁻⁹.

Dans un mode de réalisation de l'invention de procédé, le temps de vie de luminescence de la substance luminescente liée à la NANOF est augmenté, notamment par la présence de la NANOF, lorsque T_{A}/T_{L} est supérieur à 1, préférentiellement supérieur à 2, 4, 8, 10, 25, 50, 100, 1000, 10⁴, 10⁵, 10⁶, 10⁷, 10⁸, ou 10⁹.

Dans un mode de réalisation de l'invention de procédé, la variation d'au moins une propriété de luminescence de la substance luminescente associée à la NANOF par rapport à la substance luminescente seule se produit lorsque la substance luminescente possède au moins une des propriétés suivantes: (i), une énergie ou particule transférée vers la NANOF ou reçue de la NANOF, cet échange d'énergie ou de particule pouvant être en particulier un transfert de Förster ou de Dexter, (ii), une distance séparatrice la séparant de la NANOF de préférence inférieure à 1 cm, 1 µm, 500 nm, 100 nm, 50 nm, 25 nm, 10 nm, ou 5 nm, ou une distance séparatrice de préférence inférieure à 100 fois, 100 fois, 50 fois, 20 fois, 10 fois, 2 fois, ou 1 fois la distance de Förster, où la distance de Förster est de préférence comprise entre 0,1 et 50 nm, 0,5 et 20 nm, ou 1 et 10 nm, (iii), les caractéristiques d'un donneur ou d'un accepteur dans la terminologie utilisée pour décrire le transfert de Förster, (iv), la capacité d'émettre de la lumière à la(aux) longueur(s) d'onde où la NANOF absorbe de la lumière, (v), un temps de vie ou un intensité de luminescence, mesuré lorsque la substance luminescente est seule, qui est suffisamment différent de celui de la substance luminescente liée à la NANOF pour que la variation du temps de vie ou de l'intensité de luminescence puisse être mesurée, ou (vi), un temps de vie de la substance luminescente seule supérieur à 0,5 ns, 1 ns, 5 ns, 10 ns, 100 ns, 1 µs, 10 µs, 100 µs, ou 1 ms.

Dans un mode de réalisation de l'invention de procédé, la variation d'au moins une propriété de luminescence de la substance luminescente associée à la NANOF par rapport à la substance luminescente seule se produit lorsque la NANOF possède au moins l'une des propriétés suivantes: (i), une absorption, un coefficient d'absorption, un index d'absorption, ou un coefficient d'extinction significatif(s), non nul(s), préférentiellement significatif(s) à la(aux) longueur(s) d'onde d'émission de la substance luminescente, plus préférentiellement significatif(s) entre 10 et 5000 nm, 20 et 2000 nm, ou 100 et 1500 nm, (ii), un matériau contenu dans son coeur et/ou revêtement qui absorbe à la(aux) longueur(s) d'onde d'émission de la substance luminescente, (iii), une taille plus importante que celle des SPION, supérieure à 0,1 nm, 1 nm, préférentiellement supérieure à 10 nm, plus préférentiellement supérieure à 20 nm, pouvant notamment permettre d'accroître son absorption, son coefficient d'absorption, son index d'absorption, ou son coefficient d'extinction, qui peut(peuvent) augmenter avec la taille des NANOF, (iv), des propriétés ferrimagnétiques ou ferromagnétiques pouvant également permettre d'accroître l'absorption, le coefficient d'absorption, l'index d'absorption, ou le coefficient d'extinction des NANOF et/ou de favoriser le couplage entre les dipôles et/ou moments magnétiques des NANOF et ceux des substances luminescentes, ou (v), une bonne cristallinité, pouvant notamment permettre de favoriser un transfert d'énergie, de particules, préférentiellement d'électrons, entre la NANOF et la substance luminescente.

Dans un mode de réalisation de l'invention de procédé, l'atténuation de l'intensité de luminescence et/ou variation du temps de vie de luminescence de la substance luminescente associée à la NANOF par rapport à la substance luminescente seule se produit(sent) lorsque l'intensité de luminescence de la substance luminescente et le coefficient d'extinction molaire de la NANOF ainsi que la fonction de recouvrement qui leur est associée est non nulle, préférentiellement maximisée.

Dans un mode de réalisation de l'invention de procédé, l'atténuation de l'intensité de luminescence et/ou la variation du temps de vie de luminescence de la substance luminescente associée à la NANOF par rapport à la substance luminescente seule est due à la présence d'atomes d'oxygènes ou de fer dans ou à la surface de la NANOF.

Dans un mode de réalisation de l'invention de procédé, l'atténuation de l'intensité de luminescence et/ou variation du temps de vie de luminescence de la substance luminescente associée à la NANOF par rapport à la substance luminescente seule est observée lorsque la particule est activée ou inactivée. Par ailleurs, elle peut être plus prononcée lorsque la particule est inactivée que lorsqu'elle est activée.

Dans un mode de réalisation de l'invention de procédé, la dissociation de la substance luminescente de la nanoparticule d'oxyde de fer est liée à une variation de luminescence de la substance luminescente. Cela se produit préférentiellement lorsque la particule passe de l'état inactivé à l'état activé.

Dans un mode de réalisation de l'invention de procédé, la dissociation de la substance luminescente de la NANOF supprime ou diminue l'atténuation de l'intensité de luminescence, ce qui résulte en une augmentation de l'intensité de luminescence.

Dans un mode de réalisation de l'invention de procédé, la dissociation de la substance luminescente de la nanoparticule d'oxyde de fer, résulte en une variation de luminescence de la substance luminescente qui est due à : (i), un changement (début, augmentation, arrêt ou diminution) d'un transfert d'énergie ou de particule entre la substance luminescente et la NANOF, (ii), une modification de la distance séparant la substance luminescente de la NANOF, ou (iii), un changement de composition chimique ou de structure de la substance luminescence. Dans un mode de réalisation de l'invention de procédé, la dissociation de la substance luminescente de la NANOF à l'origine de la variation de luminescence est : (i), réversible en impliquant la dissociation de la substance luminescente de la NANOF suivi d'un retour à l'état initial de la particule obtenu lorsque la particule est inactivée, (ii), irréversible impliquant la dissociation de la substance luminescente de la NANOF sans retour de la substance luminescente dans son état initial, (iii), totale en impliquant l'ensemble de la substance luminescente, (iv), partielle en impliquant une partie, un groupement chimique, un atome de la substance luminescente, (v), associée à un mouvement, une translation, une oscillation, ou une vibration de la substance luminescente ou de la NANOF, (vi), associée à un couplage entre un champ électromagnétique, la substance luminescente et la NANOF, (vii), due à une variation de l'environnement de la particule, ou (viii), suivie de la diffusion de la substance luminescente ou d'une partie de cette substance dans l'environnement de la particule.

Dans un mode de réalisation de l'invention de procédé, la dissociation de la substance luminescente de la NANOF est liée à une variation locale de luminescence, se produisant lorsque la substance luminescente a diffusé à une distance, mesurée à partir du centre de la NANOF, de préférence inférieure à 100 fois, 10 fois, ou 1 fois la taille de la NANOF. Cette variation locale de luminescence est habituellement mesurée pendant un temps de mesure inférieur à 100, 10, ou 1 minute.

Dans un mode de réalisation de l'invention de procédé, au moins un paramètre lié à la dissociation de la substance luminescente tel que le nombre de substances luminescentes dissociées et/ou diffusantes, leur vitesse, ou temps de diffusion et/ou de dissociation, peut(peuvent) être mesuré(s). Il peut alors être possible d'établir une relation entre ΔL, ΔL/δt et au moins l'un de ces paramètres.

Dans un mode de réalisation de l'invention de procédé, la variation de l'environnement de la particule est liée à une variation de luminescence de la substance luminescente. Cela se produit préférentiellement lorsque la particule passe de l'état inactivé à l'état activé.

Dans un mode de réalisation de l'invention de procédé, la variation de l'environnement de la particule et/ou la pente à l'origine de cette variation peut(peuvent) être mesurée(s), avant, après, ou pendant, de préférence pendant, le fonctionnement de la particule. Il peut alors être possible d'établir une relation entre ΔL, ΔL/δt et la variation de cet environnement tel qu'une variation de pH, température, potentiel d'oxydo-réduction, composition chimique de cet environnement. Dans un mode de réalisation de l'invention de procédé, la variation du rayonnement auquel est exposée la particule selon l'invention de procédé, telle qu'une variation de l'intensité, de l'énergie, de la fréquence d'un rayonnement électromagnétique tel qu'un champ magnétique (alternatif ou non) est liée à une variation de luminescence de la substance luminescente. Cela se produit préférentiellement lorsque la particule passe de l'état inactivé à l'état activé.

Dans un mode de réalisation de l'invention de procédé, la variation du rayonnement auquel est exposée la particule selon l'invention de procédé et/ou la pente à l'origine de cette variation peut(peuvent) être mesurée(s), avant, après, ou pendant, préférentiellement pendant, le fonctionnement de la particule. Il peut alors être possible d'établir une relation entre ΔL, ΔL/δt, et la variation de ce rayonnement.

Dans un mode de réalisation de l'invention de procédé, la variation de luminescence de la sonde selon l'invention de procédé se produit lorsque ladite sonde rentre en interaction, par exemple en contact ou en collision, avec une substance, synthétique ou non synthétique. Cela peut notamment se produire lors de l'internalisation de ladite sonde dans une cellule, notamment par l'application d'un rayonnement, préférentiellement un rayonnement ionisant ou un champ magnétique.

Dans un mode de réalisation de l'invention de procédé, la variation de luminescence de la substance luminescente associée à la nanoparticule d'oxyde de fer est différente de celle de la substance luminescente seule.

Dans un mode de réalisation de l'invention de procédé, la substance luminescente possède au moins une propriété de luminescence ou subit une variation d'au moins l'une de ces propriétés qui est différente de celle de la substance luminescente seule, c'est-à-dire non liée ou non associée à la particule selon l'invention de procédé.

Dans un mode de réalisation de l'invention de procédé, la perturbation physico-chimique exercée sur la particule selon l'invention de procédé induit une variation d'au moins une propriété de luminescence de la substance luminescente qui est différente de celle induite par cette même perturbation sur la substance luminescente seule. Par exemple, une augmentation de température de l'environnement de la particule ou l'application d'un champ magnétique alternatif sur la particule peut induire la dissociation de la substance luminescente de la NANOF induisant une variation (une augmentation ou une diminution) de luminescence de la substance luminescente associée à la NANOF qui est différente de la variation de luminescence de la substance luminescente seule, non associée à la NANOF, exposée à cette même augmentation de température ou à ce même champ magnétique alternatif.

Dans un mode de réalisation de l'invention de procédé, la substance luminescente associée à la particule est un luminophore, un chromophore ou un fluorophore.

Dans un mode de réalisation de l'invention de procédé, la perturbation physico-chimique exercée sur la particule induit une augmentation ou diminution de luminescence qui est 10, 10² , 10³, 10⁶, 10¹⁰, ou 10²⁰ fois plus importante ou plus faible lorsque la substance luminescente est associée à la NANOF que lorsque la substance luminescente est seule, non associée à la NANOF..

Dans un mode de réalisation de l'invention de procédé, la perturbation physico-chimique exercée sur la substance luminescente associée à la NANOF induit une augmentation de luminescence alors que cette même perturbation induit une diminution de luminescence lorsqu'elle est exercée sur la substance luminescente seule, non associée à la NANOF.

Dans un mode de réalisation de l'invention de procédé, la perturbation physico-chimique exercée sur la substance luminescente associée à la NANOF induit une diminution de luminescence alors que cette même perturbation induit une augmentation de luminescence lorsqu'elle est exercée sur la substance luminescente seule, non associée à la NANOF.

Dans un mode de réalisation de l'invention de procédé, la perturbation physico-chimique exercée sur la substance luminescente associée à la NANOF induit une variation de luminescence, préférentiellement d'au moins 10⁻¹⁰, 10⁻⁵, 10⁻¹, 1, 10, 25, 50, 70, ou 90%, alors que cette même perturbation n'induit pas de variation de luminescence, préférentiellement une variation de luminescence de moins de 10⁻¹⁰, 10⁻⁵, 10⁻¹, 1, 10, 25, 50, 70, ou 90%, lorsqu'elle est exercée sur la substance luminescente seule, non associée à la NANOF. Dans ce cas, le pourcentage de variation de luminescence peut être défini comme étant le rapport entre la luminescence, mesurée avant et après application de la perturbation.

Dans un mode de réalisation de l'invention de procédé, la substance luminescente contient au moins un atome ou groupement chimique luminescent, une molécule luminescente, un matériau luminescent, ou un monomère ou polymère luminescent.

Dans le procédé décrit, la substance luminescente comprend ou est constituée d'au moins une substance sélectionnée dans le groupe constitué de :
ADN luminescent, ARN luminescent, Alexa fluor, Alexa fluor 350, 405, 430, 488, 500, 514, 532, 546, 548, 555, 568, 594, 610, 633, 647, 660, 669, 680, 700, 750 ou 790, une ATTO, ATTO 590, 594, 610, 611x, 620, 633, 635, 637, 647, 647N, 655, 655, 665, 680, 700, 725, 740, une Allophycocyanine (APC), une APC conjuguée, une Cy, une APC-Cy7, une aminocoumarin, une allophycocyanin (APC), APC-Cy7, une acridine orange, un 7-AAD, une azurite, AmCyan1, AcGFP1, Azami-Green, AsRed2, aminocoumarin, allophycocyanin, bromure d'éthidium bore-dipyrométhène BODIPY, BODIPY TMR, BODIPY 581/591, BODIPY TR, BODIPY-FL, BODIPY 630/650 ou 650/665, bromure d'Ethidium, une cellule luminescente, calcéine, coumarine, une cyanine Cy, Cy 2, Cy3, Cy3B, Cy 3,5, Cy 5, Cy 5,5 ou Cy 7, Cy3,5, un colorant organique, un cascade blue chromomycin A3, un cerulean, un CyPet, un "DyLight Fluor", DyLight 350, 405, 488, 549, 550, 594, 633, 649, 650, 680, 750, 755 ou 800, DRAQ5, DAPI, DCFH, DHR, dKeima-Red, Dronpa-Green, DsRed monomer, DsRed2 ("RFP"), une enzyme luminescente, une EBFP, EBFP2, ECFP, Emerald, EYFP, E2-Crimson, une fluorescéine, FluoProbe, un FluoProbe 594, 647H, 682, 752, 782, FluorX, FAM, Fluorescein FITC, un GFPuv, un hydroxycoumarin, un hoechst 33342 ou 33258, HcRed1, un HyPer, hydroxycoumarin, Hex, intercalant de l'ADN luminescent,l'iode de propidium, l'Indo-1 Fluo-3, un J-Red, un Kusabira-Orange, Katushka (TurboFP635), une luciphérase, un lipide luminescent, un lanthanide trivalent Ln³⁺, Lucifer jaune, Lissamine Rhodamine B LDS 751, un lanthanide (Lanthane, Cérium, Praséodyme, Néodyme, Prométhium, Samarium, Europium, Gadolinium, Terbium, Dysprosium, Holmium, Erbium, Thulium, Ytterbium, Lutécium), une megastokes, methoxycoumarin, mithramycin, un marqueur de fonctions cellulaires, une mCFP, mKeima-Red, mTFP1 (Teal), Midoriishi-Cyan, mCitrine, mBanana, mOrange, mOrange2, mKO, mStrawberry, mRFP1, mCherry, mKate2, mKate (TagFP635), mPlum, mRaspberry, mNeptune, Monochlorobimane, methoxycoumarin, un nanocristal semiconducteur, NBD R-Phycoerythrin (PE), une protéine luminescente, fluorescente verte, rouge ou jaune, P3, Pacific Blue, Pacific Orange, PE-Cy5, PE-Cy7, PerCP, PhiYFP, PhiYFP-m, red 613, r-phycoerythrin (PE), rhodamine red-x, rox, red 613, rhodamine, la rhodamine B, SNARF, SYTOX, SYTOX Blue, SYTOX Green, SYTOX Orange, S65A, S65C, S65L, S65T, une substance luminescente contenue dans un kit d'étiquetage par anticorps tel un "Biotin Tag", "Fluoro Tag", "Immuno Probe" ou "Mix-n-Stain", un tissu luminescent, un Texas Red, une TOTO-3, TO-PRO-3, TruRed, TRITC X-Rhodamine, TOTO-1, TO-PRO-1, TO-PRO: Cyanine : Monomer, Thiazole Orange, TOTO-3, TO-PRO-3, T-Sapphire, TagBFP, TagCFP, TurboGFP, TagGFP, TagGFP2, Topaz, TurboYFP, TurboRFP, tdTomato DsRed-Express2, TagRFP, TurboFP602, TurboFP635, TRITC, Tamara, Texas Red, TruRed, Venus, YOYO-1 Y66H, Y66F, Y66W, YPet, ZsGreen1, ZsYellow1, et une substance dérivée des substances luminescentes précitées ou ayant au moins un atome, une molécule, un groupement chimique luminescent en commun avec les substances luminescentes précitées.

Dans l'invention de procédé, la particule selon l'invention de procédé est associée à une substance luminescente qui possède au moins une fonction chimique sélectionnée dans le groupe constitué des fonctions acide carboxylique, phosphorique, sulfonique esters, amide, cétone, alcool, phénol, thiol, amine, éther, sulfure, anhydride d'acide, halogénure d'acyle, amidine, nitrile, hydropéroxyde, imine, aldéhyde, peroxyde.

Dans un mode de réalisation de l'invention de procédé, la substance luminescence possède au moins une fonction chimique qui permet une liaison faible avec la nanoparticule d'oxyde de fer, telle qu'une fonction acide carboxylique.

Dans un mode de réalisation de l'invention de procédé, la perturbation physico-chimique est exercée sur la particule par un rayonnement, préférentiellement un champ magnétique, plus préférentiellement un champ magnétique alternatif.

Dans un mode de réalisation de l'invention de procédé, le rayonnement est tel qu'il modifie de manière significative la NANOF, tel que cela est défini dans l'invention.

Dans un autre mode de réalisation de l'invention de procédé, le rayonnement est un champ magnétique continu, d'intensité préférentiellement comprise entre 0,01 mT et 100 T, 0,1 mT et 10 T, ou 1 mT et 1 T.

Dans un mode de réalisation de l'invention de procédé, le rayonnement est un champ magnétique alternatif d'intensité préférentiellement comprise entre 0,01 mT et 100 T, 0,1 mT et 10 T, ou 1 mT et 1 T et de fréquence préférentiellement comprise entre 0,001 Hz et 1000 MHz, 0,01 Hz et 100 MHz, 0,1 Hz et 10 MHz, 1 Hz et 1 MHz, 10 Hz et 500 kHz, 100 Hz et 500 kHz, 1 kHz et 250 kHz, ou 10 kHz et 200 kHz..

Dans un mode de réalisation de l'invention de procédé, la perturbation physico-chimique exercée sur la particule ou la variation de l'environnement de la particule selon l'invention de procédé est une variation de pH de moins de 14, 13, 12, 11, 10, 9, 8, 7, 6, 5, 4, 3, 2, 1, 0,5, 0,2, 0,1, 0,01, 0,005, 0,001, 10⁻⁴, 10⁻⁵, 10⁻⁶, 10⁻⁷, 10⁻⁸, 10⁻⁹, 10⁻¹⁰, 10⁻¹¹ 10⁻¹², ou 10⁻¹³ unités de pH de cet environnement ou d'au moins une substance contenue dans cet environnement.

Dans un mode de réalisation de l'invention de procédé, la perturbation physico-chimique exercée sur la particule ou la variation de l'environnement de la particule selon l'invention de procédé est une variation de pH de plus de 14, 13, 12, 11, 10, 9, 8, 7, 6, 5, 4, 3, 2, 1, 0,5, 0,2, 0,1, 0,01, 0,005, 0,001, 10⁻⁴, 10⁻⁵, 10⁻⁶, 10⁻⁷, 10⁻⁸, 10⁻⁹, 10⁻¹⁰, 10⁻¹¹, 10⁻¹², ou 10⁻¹³ unités de pH de cet environnement ou d'au moins une substance contenue dans cet environnement.

Dans un mode de réalisation de l'invention de procédé, la perturbation physico-chimique exercée sur la particule ou la variation de l'environnement de la particule selon l'invention de procédé est une variation de température de moins de 10¹⁰, 10⁹, 10⁸, 10⁷, 10⁶, 10⁵, 10⁴, 10³, 500, 250, 150, 100, 50, 40, 20, 30, 10, 5, 1, 0,1, 0,01, 0,001, 10⁻⁴, 10⁻⁵, 10⁻⁶, 10⁻⁷, 10⁻⁸, 10⁻⁹, 10⁻¹⁰, 10⁻¹¹, 10⁻¹², ou 10⁻¹³ °C de cet environnement ou d'au moins une substance contenue dans cet environnement.

Dans un mode de réalisation de l'invention de procédé, la perturbation physico-chimique exercée sur la particule ou la variation de l'environnement de la particule selon l'invention de procédé est une variation de température de plus de 10¹⁰, 10⁹, 10⁸, 10⁷, 10⁶, 10⁵, 10⁴, 10³, 500, 250, 150, 100, 50, 40, 20, 30, 10, 5, 1, 0,1, 0,01, 0,001, 10⁻⁴, 10⁻⁵, 10⁻⁶, 10⁻⁷, 10⁻⁸, 10⁻⁹, 10⁻¹⁰, 10⁻¹¹, 10⁻¹², ou 10⁻¹³ °C de cet environnement ou d'au moins une substance contenue dans cet environnement.

Dans un mode de réalisation du procédé *in vitro* de détection de variation de luminescence selon l'invention, la variation de luminescence est mesurée durant un intervalle de temps inférieur à une heure, une minute, ou une seconde.

Dans un autre mode de réalisation du procédé *in vitro* de détection de variation de luminescence selon l'invention, la variation de luminescence est mesurée localement autour de la particule, de préférence à une distance mesurée à partir du centre de la particule inférieure à 1 cm³, 1 mm³, ou 0,1 mm³.

Dans un autre mode de réalisation du procédé *in vitro* de détection de variation de luminescence selon l'invention, la variation de luminescence est une augmentation continue de luminescence de la substance luminescente, préférentiellement durant au moins une seconde, une minute, ou une heure.

Dans un autre mode de réalisation du procédé *in vitro* de détection de variation de luminescence selon l'invention, on détecte la dissociation de la substance luminescente de la NANOF.

Dans un autre mode de réalisation de l'invention de procédé, le procédé de détection de la variation de luminescence selon l'invention est appliqué pour détecter au moins un paramètre physiologique, préférentiellement la température, le pH, l'environnement de la nanoparticule d'oxyde de fer.

Dans un autre mode de réalisation, le procédé de détection de la variation de luminescence selon l'invention est appliqué pour détecter un rayonnement tel que défini ci-dessus.

Dans un autre mode de réalisation du procédé *in vitro* de détection de variation de luminescence selon l'invention, l'intensité de luminescence de la particule selon l'invention est augmentée par application d'un rayonnement tel que défini ci-dessus.

Dans un autre mode de réalisation du procédé *in vitro* de détection de variation de luminescence selon l'invention, l'intensité de luminescence de la substance luminescente augmente par variation de l'environnement de la particule.

Dans un mode de réalisation de l'invention de procédé, la particule selon l'invention de procédé peut(peuvent) servir à mesurer la présence de la particule et sa distribution spatiale. Dans ce cas, un rayonnement tel que défini ci-dessus peut être utilisé pour accroître la luminescence de la particule et la rendre plus facilement détectable par le dispositif selon l'invention de procédé. Par ailleurs, un système optique comprenant une fibre optique que l'on déplace horizontalement au-dessus d'un échantillon contenant la particule selon l'invention de procédé peut être utilisé pour mesurer la distribution spatiale de la particule. Il peut alors être possible d'établir une relation entre une variation spatiale de luminescence de la particule et sa distribution dans un milieu donné.

Dans un mode de réalisation de l'invention de procédé, la substance luminescente a une taille au moins 2, 5, 10, 10², 10³, 10⁴, 10⁵, ou 10¹⁰ fois inférieure à la taille de la nanoparticule, ce qui peut permettre de lier ou d'associer un nombre important de substances luminescentes à la nanoparticule.

Dans un mode de réalisation de l'invention de procédé, la substance luminescente a une petite taille, préférentiellement inférieure à 10 ou 1 nm, 100, 10, 5, 1 ou 0,1 Å, ou un petit poids moléculaire, préférentiellement inférieur à 10⁶, 10⁵, 10⁴, 10³, 10², ou 10 g/mol, ce qui peut permettre de lier ou d'associer un nombre important de substances luminescentes à la nanoparticule.

Dans un mode de réalisation de l'invention de procédé, la substance luminescente est liée ou associée à la nanoparticule par au moins une substance dont la taille est inférieure à 10 ou 1 nm, 100, 10, 5, 1 ou 0,1 Å, ou dont le poids moléculaire est inférieur à 10⁶, 10⁵, 10⁴, 10³, 10², ou 10 g/mol, ce qui peut permettre de lier ou d'associer un nombre important de substances luminescentes à la nanoparticule.

Dans un mode de réalisation de l'invention de procédé, on préfère éviter l'utilisation de certaines structures de grande taille, d'une taille supérieure à 10 ou 1 µm, 100, 10 ou 1 nm, 100, 10, 5, 1 ou 0,1 Å, telles que des substances biologiques, protéines, lipides, ADN, ARN, ou de grosses molécules telles que des dendrimères, pour lier ou associer la substance luminescente à la nanoparticule. Ce type de structure peut effet d'une part limiter le nombre de substances luminescentes que l'on peut lier ou associer à la nanoparticule et d'autre part créer une distance importante, préférentiellement supérieure à 10 ou 1 µm, 100 ou 10 nm, entre la substance luminescente et la nanoparticule, et ainsi gêner le fonctionnement de la sonde ou de la particule, notamment l'atténuation de luminescence de la substance luminescente lorsque la substance luminescente est à proximité de la nanoparticule.

Dans un mode de réalisation de l'invention de procédé, le rapport surface/volume de la nanoparticule est suffisamment élevé pour permettre l'association du composé ou de la substance luminescente, est préférentiellement supérieur à 10⁻¹⁰, 10⁻⁵, 10⁻³, 10⁻¹, 0,5, ou 0,9 nm⁻¹.

Dans un autre mode de réalisation de l'invention de procédé, le rapport surface/volume de la nanoparticule n'est pas trop élevé pour éviter que la nanoparticule ne perde ses propriétés ferrimagnétiques ou ferromagnétiques, est préférentiellement inférieur à 10³, 10², 10, 1, 0,1, ou 0,01 nm⁻¹.

Dans un mode de réalisation de l'invention de procédé, au moins 2, 5, 10, 10², 10³, 10⁴, 10⁵, ou 10¹⁰ substances luminescentes sont associées ou liées à la nanoparticule ou à chaque nanoparticule, préférentiellement à chaque nanoparticule en moyenne. Dans certains cas, le nombre de substances luminescentes associées ou liées à chaque nanoparticule en moyenne peut être estimé en mesurant le rapport entre de nombre de substances luminescentes non associées à la nanoparticule contenues dans un volume donné, mesuré lorsque la particule est activée ou inactivée, préférentiellement lorsque la particule est activée, divisé par le nombre de nanoparticules non associées à la substance luminescente contenu dans le même volume, mesuré lorsque la particule est activée ou non activée, préférentiellement lorsque la particule est activée.

Dans un mode de réalisation de l'invention de procédé, la particule comprenant la substance luminescente associée ou liée à la nanoparticule possède une SAR, une coercivité, un rapport entre aimantation rémanente et aimantation à saturation, ou aimantation à saturation qui est(sont) au moins égal ou égaux à 10⁻⁴, 10⁻³, 10⁻², 10⁻¹, 1, 1,001, 1,01, 1,2, 1,5, 2, 5, 10, ou 100 que multiplie la SAR, la coercivité, rapport entre aimantation rémanente et aimantation à saturation, ou aimantation à saturation de la nanoparticule respectivement.

Dans un mode de réalisation de l'invention de procédé, au moins deux des environnements suivant peuvent être équivalents : l'environnement chimique, l'environnement de la particule, l'environnement de la NANOF, l'environnement du composé, l'environnement de la sonde, l'environnement naturel de la particule, l'environnement radioactif, l'environnement biologique.

### DESCRIPTION DE LA FIGURE

**Figure 1** : Figure schématique indiquant la fabrication et le fonctionnement de la particule. Sous l'effet d'un rayonnement ionisant ou d'une variation de l'environnement de la particule, le composé se dissocié de la NANOF et/ou se modifie chimiquement.

### EXEMPLES EXPERIMENTAUX :

### Signification des abréviations.

Dans la description des exemples expérimentaux, MC désigne des chaînes de magnétosomes extraites des bactéries magnétotactiques non luminescentes cultivées en absence de rhodamine B, MCR400 et MCR20 désignent des chaînes de magnétosomes extraites de bactéries magnétotactiques, où les bactéries sont synthétisées en présence de 400 µM de rhodamine B et 20 µM de rhodamine B. BNF-Dil et MC-Dil désignent respectivement des BNF-Starch plain (Micromod 10-00-102) et magnétosomes en chaînes préparés en présence de DiI (Sigma 42364 ; 1,1'-Dioctadecyl-3,3,3',3'-tetramethyl-indocarbocyanine perchlorate). Le surnageant des MCR400, BNF-Dil et MC-Dil est le liquide retiré après avoir attiré les différentes nanoparticules d'oxyde de fer en suspension à l'aide d'un aimant au Néodinium de 0,6 T.

### Exemple 1: Synthèse et caractéristiques des MC et MCR400.

Les suspensions de MCR400 et de MC ont été préparées selon le protocole expérimental suivant : Les bactéries magnétotactiques *Magnetospirillum magneticum* AMB-1 ont été obtenues à l'ATCC (ATCC 700274) et ont été cultivées dans des conditions micro-anaérobiques à la température de 30°C dans un milieu de culture liquide légèrement modifié par rapport au milieu MSGM (Milieu ATCC 1653). Dans un litre, ce milieu de culture contient 0,68 g de potassium phosphate monobasique, 0,85 g de succinate de sodium, 0,57 g de tartrate de sodium, 0,083 g d'acétate de sodium, 225 µl de résazurine à 0,2%, 0,17 g de nitrate de sodium, 0,04 g d'acide L-ascorbique, 2 ml d'une solution de quinate de fer à 10 mM, 10 ml d'une solution de vitamines de Woolf et 5 ml d'une solution de minéraux de Woolf. Pour préparer les MC, aucune solution de rhodamine n'est ajoutée au milieu de culture. Pour préparer les MCR400, 100 mL d'une solution de rhodamine B 4 mM sont introduits dans le milieu de culture des bactéries. Pour préparer les MCR20, 5 mL d'une solution de rhodamine B 4 mM sont introduits dans le milieu de culture des bactéries. Le pH du milieu de culture est ajusté à 6,85 en utilisant une solution de soude à 1M. Les bactéries sont collectées pendant la phase stationnaire et les bactéries sont concentrées à l'aide d'une colonne (mPES, 500KDa) de filtration à flux tangentielle avec un débit de 950 mL/min puis lavées 5 fois pendant 30 minutes avec une solution de tampon phosphate salin à pH 7,4 (137 mM NaCl, 2,7 mM KCl, 10 mM Na₂HPO₄, 1,76 mM KH₂PO₄). Les bactéries sont collectées par centrifugation à 4000 tours par minute pendant 1 heure, le surnageant est retiré et les bactéries sont re-suspendues dans 50 mM d'une solution tampon de Tris-HCl à pH 7,4 et diluées jusqu'à l'obtention d'une densité optique de 5 à 600 nm. Afin d'extraire les chaînes de magnétosomes, cette suspension est soniquée à 5°C pendant 60 minutes à 30 W (avec un pulse de 2 sec et un intervalle entre pulses de 1 sec) pour lyser les membranes cellulaires, isolant ainsi les chaînes de magnétosomes des bactéries. Après sonication, les chaînes de magnétosomes sont isolées magnétiquement des débris cellulaires à l'aide d'un aimant de néodyme. Le surnageant contenant les débris cellulaires est retiré et les chaînes de magnétosomes sont lavées cinq fois magnétiquement à l'aide d'une solution tampon de Tris-HCl 50 mM à pH 7,4 puis quinze fois avec de l'eau Millipore^{®}. Elles sont finalement resuspendues dans de l'eau stérile Millipore^{®} pour obtenir une suspension de MCR400, de MCR20 ou de MC.

L'association de la rhodamine B aux MCR400 est mise en évidence par le spectre d'absorption d'une suspension de MCR400 à une concentration en fer de 40µg/mL. Ce spectre présente un épaulement à 550 nm, absent dans le spectre d'absorption des MC et correspondant à la longueur d'onde d'absorption maximale de la rhodamine B. Par ailleurs, le surnageant de la suspension des MCR400 absorbe peu à la longueur d'onde d'absorption de la rhodamine B. Ceci confirme que l'absorption de la rhodamine B dans la suspension de MCR400 provient de la rhodamine B associée aux magnétosomes. Cette association peut aussi être révélée par les spectres d'émission de fluorescence des MCR400. En effet, la suspension de MCR400, excitée à 405 nm, a un maximum d'intensité de fluorescence à 569 nm, une longueur d'onde légèrement inférieure à celle correspondant à l'intensité maximum de fluorescence de la rhodamine B qui est de 576 nm. Cette différence pourrait s'expliquer par l'association de la rhodamine B et des chaînes de magnétosomes qui modifierait légèrement la longueur d'onde d'émission des MCR400 par rapport à celle de la rhodamine B. Par ailleurs, le spectre infrarouge des MCR400 que nous avons mesuré révèle la présence de bande de déformation et de vibration caractéristiques des MC (Amide I à 1650 cm⁻¹, amide II à 1530 cm⁻¹, lipopolysaccharides (LPS) ou phospholipides à 1050 cm⁻¹ et à 1250 cm⁻¹, maghémite à 580 cm⁻¹) ainsi que ceux caractéristiques de la rhodamine B (acide carboxylique à 1700 cm⁻¹, alcène à 1475 cm⁻¹ et à 680 cm⁻¹, éther à 1250 cm⁻¹ et à 1175 cm⁻¹, amine III à 1125 cm⁻¹). Ces résultats semblent donc confirmer que la rhodamine B est soit adsorbée à la surface des magnétosomes soit complexée aux magnétosomes, possiblement grâce à la fonction acide carboxylique de la rhodamine B. Une expérience a également permis de déterminer le nombre de molécules de rhodamine B présentes sur ou dans chaque magnétosome. Pour cela, les MCR400 ont été mis en présence d'acide chlorhydrique à pH = 2 afin de libérer la rhodamine B des magnétosomes et de transformer l'oxyde de fer en ions fer (III) et fer (II). Le spectre de fluorescence des MCR400 ainsi traités suggère que la rhodamine B est bien libérée des magnétosomes puisque le pic de luminescence est à 582 nm (la longueur d'onde d'émission de la rhodamine B libre en solution à pH 2) et non pas à 568 nm (la longueur d'onde d'émission de la rhodamine B associée aux magnétosomes dans les MCR400). A partir de l'intensité de luminescence de la rhodamine B libérée des magnétosomes (~ 0.56 u. a.), une concentration en rhodamine B de 750 nmol/L est estimée à partir d'une courbe d'étalonnage représentant la variation de luminescence de la rhodamine B seule en solution à pH = 2 en fonction de la concentration en rhodamine B. Dans 1 mL, il y a donc 4,6 10¹⁴ molécules de RhB. Connaissant la concentration des magnétosomes en suspension de ~166 µg/mL et la masse d'un magnétosomes de 6,25 10⁻¹⁶ g, on en déduit le nombre de magnétosomes dans 1 mL comme étant 26,5 10¹⁰ magnétosomes et le nombre de molécules de rhodamine B associées aux magnétosomes dans les MCR400 est estimé à ~178±4. En d'autres termes, 178±4 molécules de rhodamine B sont associées à chaque magnétosome en moyenne.

### Nous pouvons conclure de cet exemple que :

(i) Nous avons présenté une méthode de fabrication des MCR400.
(ii) Nous avons mis en évidence l'association de la rhodamine B aux magnétosomes dans les MCR400.
(iii) Les MCR400 sont caractérisés par un pic d'absorption de longueur d'onde similaire à celle de la rhodamine B et par un pic d'émission à une longueur d'émission différente de celle de la rhodamine B d'environ 7-10 nm.

### Exemple 2: Synthèse et propriétés des MC-Dil et BNF-Dil.

Les suspensions de MC-Dil et BNF-Dil sont préparées, respectivement à partir d'une suspension de MC et de BNF-starch, préalablement concentrée à 25 mg/mL en oxyde de fer par centrifugation à 14 000 tours par minutes pendant 30 minutes à 5°C. Une solution stock de DiI à 5 mg/mL est d'abord préparée dans de l'éthanol absolu préalablement distillé. 5 mg d'oxyde de fer des suspensions de MC et de BNF-starch sont ensuite introduits dans 200µL d'éthanol absolu distillé puis mélangés à 2,5 mg de DiI provenant de la solution stock. Le mélange est soniqué à 30W pendant 10 min (pulse de 2 sec.), à l'abri de la lumière et à une température comprise entre 5 et 10 °C. Un volume d' 1mL d'eau stérile Millipore^{®} est ajouté à la suspension soniquée puis l'échantillon est laissé sous agitation magnétique pendant 1h à température ambiante et à l'abri de la lumière. Par la suite, ce mélange est conservé à 5 °C pendant 24 heures. Le mélange est soniqué à 30W pendant 10 min (pulse 2 sec.) à l'abri de la lumière et à une température comprise entre 5 et 10 °C. Les suspensions de MC-Dil et BNF-Dil sont concentrées par centrifugation à 14 000 tours par minutes pendant 30 minutes à 5°C puis lavées cinq fois à l'éthanol 0,5% et quinze fois à l'eau stérile Millipore^{®}. Elles sont finalement re-suspendues dans de l'eau stérile Millipore^{®} pour obtenir des suspensions de MC-Dil ou de BNF-DiI.

Les spectres d'absorption du DiI présentent deux maximums à 560 nm et 520 nm. Les BNF-DiI présentent trois pics d'absorption à des longueurs d'onde différentes à 595 nm, à 535 nm et à 490 nm. Le surnageant des BNF-DiI n'absorbe pas aux longueurs d'onde d'absorption du DiI. Les spectres d'émission du DiI excité à 530 nm présentent deux pics d'intensité maximale à 640 nm et 591 nm. Comme pour les spectres d'absorption, un décalage spectral est observé entre les spectres d'émission du BNF-Dil excité à 530 nm dont les pics d'intensité maximale sont à 670 nm et 600 nm et ceux du DiI. Par ailleurs, le surnageant des BNF-DiI n'émet pas d'intensité de luminescence aux longueurs d'onde d'émission du DiI. Les décalages spectraux observés en absorption et émission entre BNF-DiI et DiI suggèrent une modification du DiI lorsqu'il est associé aux BNF. L'absence d'absorption et d'émission du surnageant des BNF-DiI suggère l'absence de DiI dans ce surnageant et l'association du DiI aux BNF dans les BNF-DiI.

Les MC-Dil présentent un comportement relativement similaire aux BNF-DiI avec deux pics d'absorption à 570 nm et 520 nm, deux pics d'émission à 680 nm et 600 nm. Comme pour les BNF-DiI, les différences observées entre les pics d'absorption et d'émission du MC-Dil et ceux du DiI suggèrent une modification du DiI lorsqu'il est associé aux MC. L'absence d'absorption et d'émission du surnageant des MC-Dil suggère l'association du DiI aux MC dans les MC-DiI.

### Nous pouvons conclure de cet exemple que :

(i) Nous avons présenté une méthode de fabrication des BNF-DiI et des MC-Dil
(ii) Nous avons mis en évidence l'association du DiI aux BNF et aux MC.
(iii) Les BNF-Dil et MC-Dil sont caractérisés par des pics d'absorption et d'émission dont les longueurs d'onde sont différentes de celles du DiI.

### Exemple 3 : Utilisation des MCR400 comme sonde de température.

Une solution de rhodamine B de concentration 5 µM et de pH 7 contenue dans un volume de 800 µL, est excitée à 405 nm et chauffée à différentes températures pendant 30 minutes à l'aide d'un bain chauffant à sec. Lorsque la température de cette solution augmente de 20 °C à 80 °C, la longueur d'onde correspondant au maximum de luminescence de la rhodamine B reste relativement inchangée alors que l'intensité maximale de luminescence de rhodamine B diminue fortement de 73% en accord avec des résultats précédemment publiés (Journal of Luminescence, Vol. 27, P. 455 (1982)). Une suspension de MCR400 de concentration 250 µg/mL en oxyde de fer et de pH 7, contenue dans un volume de 800 µl, est excitée à 405 nm et chauffée à différentes températures pendant 30 minutes à l'aide d'un bain chauffant à sec. Les MCR400 excités à 405 nm produisent une intensité maximale de luminescence à 569 nm. Le comportement des MCR400 est différent de celui de la rhodamine B. En effet, entre 20°C et 80°C, le maximum du pic de luminescence est observé à 569 nm et l'intensité de luminescence des MCR400 augmente fortement de 66%. L'intensité de luminescence de la rhodamine B serait initialement atténuée lorsque la rhodamine B est associée aux magnétosomes et augmenterait avec la dissociation de la rhodamine B des magnétosomes sous l'effet d'une augmentation de température.

La dissociation de la rhodamine B des MCR400 par chauffage est confirmée par des mesures d'absorption. Des suspensions de MCR400 de concentration 40 µg/mL en oxyde de fer sont chauffées pendant une période de 0 à 240 minutes à pH 7 et l'absorption du surnageant des MCR400 est mesurée à 550 nm. Les variations de l'absorption du surnageant des MCR400 sont mesurées en fonction du temps et de la température de chauffage. Plus la suspension de MCR400 est chauffée longtemps ou à une température élevée, plus l'absorption du surnageant est élevée. A 20 °C, l'absorption du surnageant des MCR400 augmente de 0 à ~0.004 en 240 minutes. A 60 °C, l'absorption du surnageant augmente de ~0,01 à ~0,028 en 240 minutes. A 90 °C, l'absorption du surnageant augmente de ~0,022 à ~0,044 en 240 minutes. Lorsque les MSR400 sont chauffés à différentes températures pendant 240 minutes, la concentration en rhodamine B dans le surnageant des MCR400 augmente de 20 nM à 20 °C à 560 nM à 90 °C.

### Nous pouvons conclure de cet exemple que :

(i) Il est possible d'établir une relation entre la variation de l'intensité de luminescence et la variation de température des MCR400 et donc d'utiliser les MCR400 comme sonde de température.
(ii) L'intensité de luminescence des MCR400 augmente avec l'augmentation de température, un comportement opposé à celui de la rhodamine B libre en solution. Cela peut notamment permettre de distinguer entre la présence de rhodamine B libre ou de rhodamine B associée aux magnétosomes.
(iii) L'augmentation de luminescence des MCR400 avec la température peut être attribuée à la dissociation de la rhodamine B des MCR400 lorsque ces derniers sont chauffés.
(iv) La quantité de rhodamine B dissociée des magnétosomes par chauffage peut être évaluée par des mesures d'absorption.

### Exemple 4 : Utilisation des MCR400 comme sonde de détection d'irradiation.

Trois suspensions, maintenues à pH 7 et à une température de 25 °C, contenant soit des MCR400 de concentration 400 µg/mL en oxyde de fer, soit de la rhodamine B à 125µM, soit des MC à 400 µg/mL en oxyde de fer mélangés à de la rhodamine B à 125µM. Ces suspensions sont irradiées à l'aide d'un dosimètre irradiateur faxitron (160 kV, 6,3 mA et sans filtre, 67,5 Gy/min). Les doses d'irradiation sont comprises entre 0 et 1350 Gy. La luminescence, excitée à 550 nm et mesurée à 578 nm, des suspensions contenant la rhodamine B seule ou le surnageant du mélange de rhodamine B et de MC ne varie pas ou diminue lorsque ces suspensions sont irradiées. Par contraste, lorsque la suspension de MCR400 est irradiée, la luminescence du surnageant de cette suspension, excitée à 550 nm et mesurée à 576 nm, augmente fortement de 250 u. a. en absence d'irradiation à 600-950 u. a. en présence d'une irradiation supérieure à 250 Gy. Ces résultats suggèrent la dissociation de la rhodamine B des MCR400 sous l'effet d'une irradiation. Par ailleurs, la longueur d'onde d'émission du surnageant des MCR400 et de la rhodamine B libre en solution reste inchangée à 576 nm, et ce quelle que soit la dose d'irradiation reçue par les MCR400 ou la rhodamine B. Ceci suggère que la rhodamine B libre en solution ou associée aux magnétosomes n'a pas été modifiée sous l'effet de l'irradiation.

### Nous pouvons conclure de cet exemple que :

(i) Il est possible d'établir une relation entre la variation de luminescence et la variation d'irradiation des MCR400 et donc d'utiliser les MCR400 comme sonde d'irradiation.
(ii) L'intensité de luminescence du surnageant des MCR400 augmente avec l'augmentation de l'irradiation, un comportement différent de celui de la rhodamine B libre en solution. Cela peut notamment permettre de distinguer entre la présence de rhodamine B libre ou de rhodamine B associée aux magnétosomes.
(iii) L'augmentation de luminescence du surnageant des MCR400 avec l'irradiation peut être attribuée à la dissociation de la rhodamine B des MCR400 lorsque ces derniers sont irradiés.
(iv) La quantité de rhodamine B dissociée des magnétosomes par irradiation peut être évaluée par des mesures de luminescence.

### Exemple 5 : Utilisation des BNF-Dil comme sonde de température.

Une suspension de DiI de concentration à 5,4 µM et à pH 7 est chauffée à différentes températures comprises entre 20 et 90 °C pendant 30 minutes à l'aide d'un bain chauffant à sec. L'intensité maximale de luminescence du DiI, excité à 530 nm, augmente entre 20 et 60 °C puis diminue entre 60 et 100°C. Lorsqu'une suspension contenant des BNF-Dil mélangée dans l'eau à une concentration de 80 µg/mL en oxyde de fer est chauffée à des températures comprises entre 20 et 90 °C à l'aide d'un bain chauffant à sec, le comportement est différent de celui observé avec le DiI seul. En effet, l'intensité maximale de luminescence des BNF-Dil, excités à 530 nm, diminue entre 20 et 60°C puis reste quasi stable entre 60 et 100 °C.

Afin de déterminer si la variation de luminescence des BNF-DiI est due à une dissociation du DiI des BNF, l'intensité maximale de luminescence du surnageant des BNF-Dil et des DiI seuls, excitées à 530 nm, ont été mesurées pour des suspensions contenant les BNF-DiI ou les DiI seuls chauffées à différentes températures comprises entre 20 et 90 °C pendant une période de 0 à 30 minutes. L'intensité de luminescence du surnageant des BNF-DiI n'augmente pas lorsque les BNF-DiI sont chauffés entre 20 et 90 °C pendant une période de 0 à 30 minutes. Sachant que lorsque le DiI est chauffé entre 20 et 90 °C pendant une période de 0 à 30 minutes, son intensité de luminescence ne diminue pas de manière significative, cela suggère que la concentration du DiI dans le surnageant des BNF-Dil n'augmente pas lorsque la température augmente et que le DiI ne se dissocie donc pas des BNF.

### Nous pouvons conclure de cet exemple que :

(i) Il est possible d'établir une relation entre la variation de l'intensité de luminescence et la variation de température des BNF-Dil et donc d'utiliser les BNF-Dil comme sonde de température.
(ii) L'intensité de luminescence du surnageant des BNF-Dil n'augmente pas avec l'augmentation de température, ce qui suggère que le DiI ne se dissocie pas des BNF sous l'effet d'une augmentation de température. Dans ce cas, la diminution de la luminescence des BNF-DiI avec l'augmentation de température pourrait s'expliquer par une modification chimique des BNF-DiI (exemple illustratif seulement).

### Exemple 6 : Utilisation des MCR400 comme sonde de pH.

Des MCR400 sont testés comme sonde de pH *in vivo* sur des cerveaux de rats. Pour cela, 4 rats sont utilisés. Le rat 1 est un rat sain euthanasié en même temps que le rat 2. Les rats 2 à 4 reçoivent 5 µL d'une suspension contenant 3×10³ cellules RG-2, implantés à l'aide d'un casque de stéréotaxie aux coordonnées 2 mm antérieur, 2 mm latéral et 4 mm de profondeur. Le rat 2 ne subit pas de traitement et est euthanasié 18 jours après l'implantation des cellules. Les rats 3 et 4 reçoivent quatorze jours après l'implantation des cellules tumorale à l'endroit de cette implantation 10 µL d'une suspension de MCR400 à une concentration de 668 µg/mL en oxyde de fer. Les rats 3 et 4 sont euthanasiés 2 heures et 4 jours après l'administration des magnétosomes respectivement. Les cerveaux sont ensuite extraits puis découpés en couches de 3 mm d'épaisseur. La luminescence des tranches est ensuite produite à l'aide d'une diode laser pulsée de 40MHz avec une puissance de 1mW et un rayonnement à 405 nm. La lumière du laser est couplée à une fibre optique de diamètre de 200 µm positionnée à 1,5 mm au-dessus des tranches de cerveau. La lumière émise par le tissu est collectée par une deuxième fibre optique de diamètre 365 µm située à 600 µm de la première fibre et positionnée 1,5 mm au-dessus des tranches de cerveau. La luminescence est détectée par un spectromètre (B. Leh et al., J. Biomed. Opt. (2012) 17(10), 108001) permettant de mesurer l'intensité de luminescence des tranches de cerveau en fonction de la longueur d'onde du rayonnement émis. Les deux fibres optiques peuvent être déplacées latéralement à différents endroits des tranches de cerveau pour repérer les zones de luminescence.

Pour les différents rats, les spectres de luminescence des zones saines ne contenant pas de MCR400 ont été mesurés, sont très similaires, une bande de luminescence avec un maximum d'intensité a été observée à environ 500 nm. Cette bande serait due à la contribution minoritaire de la luminescence du NADH à 450 nm et à celle dominante des flavoprotéines à 525 nm. A 575 nm, un épaulement est observé qui proviendrait de la luminescence des lipo-pigments appelés aussi lipofucine débris lipidiques. A 625 et 690 nm, la luminescence de la protoporphyrine IX et d'une molécule en dérivant sont observés. Les spectres des zones saines ont été moyennés permettant ainsi de normaliser tous les spectres de luminescence mesurés pour toutes les tranches de cerveau de rat.

Pour les différents rats, les spectres de luminescence des zones tumorales ne contenant pas de MCR400 ont également été mesurés, sont très similaires, indiquant la présence de pics similaires à ceux observés pour la zone saine avec de plus faibles intensités.

Pour le rat 3 euthanasié deux heures après administration des MCR400, le spectre de luminescence de la zone tumorale ayant reçu les MCR400 indique la présence de deux pics intéressants, l'un à 569 nm qui n'est pas présent dans les spectres des zones tumorales et saines ne contenant pas les MCR400 et l'autre à 576 nm. Nous attribuons ces deux pics à l'émission des MCR400 à 569 nm et à celle de la rhodamine B et des lipo-pigments à 576 nm. Le pic à 576 nm ne peut pas uniquement provenir de la luminescence des lipo-pigments, car son intensité relative de luminescence, I₅₇₆/I₅₀₀ ~ 1, est plus élevée que celle de I₅₇₆/I₅₀₀ ~ 0.5 mesurée à partir des spectres de luminescence des zones saines et tumorales ne contenant pas les MCR400, où I₅₇₆ et I₅₀₀ sont les intensités de luminescence mesurées à 576 nm et à 500 nm respectivement. Pour le rat 4 euthanasié quatre jours après administration des MCR400, le spectre de luminescence de la zone tumorale ayant reçu les MCR400 indique la présence du pic provenant de la rhodamine B et des lipo-pigments à 576 nm. Le pic à 569 nm provenant des MCR400 n'est plus présent. Ceci pourrait s'expliquer par la dissociation de la rhodamine B des MCR400, suite à l'administration des MCR400 dans le cerveau des rats.

Afin d'étudier si cette dissociation est due à une variation de pH, on étudie d'abord si les MCR400 s'internalisent dans des cellules viables, ce qui pourrait résulter en une variation de pH. Des MCR400 et 500 000 cellules MDA-MB-231 sont mises en présence d'une sonde de viabilité cellulaire, la calcéine-AcétoxyMéthylester (Ca) qui n'est pas naturellement luminescente et qui luminesce en présence de cellules vivantes. Les cellules sont incubées durant 6 heures soit en présence de Ca soit en présence de Ca et MCR400 où la concentration en oxyde de fer des suspensions de MCR400 est de 31,5 µg/mL. Le cytomètre en flux permet de détecter soit la luminescence de Ca (Signal FL1-H), soit celle de la rhodamine B (signal FL3-H). Le signal FL1-H est faible à 2-3 pour les cellules seules et augmente à ~ 100 lorsque les cellules sont en présence de Ca indiquant la viabilité des cellules MDA-MB-231 en absence de MCR400. Lorsque les cellules sont incubées en présence de Ca et MCR400, le signal FL1-H est supérieur à ~ 10 et la majorité des cellules sont donc viables. Quant au signal FL3-H, il indique que 99 % des cellules sont luminescentes en présence de MCR400. Afin d'étudier l'internalisation des MCR400, les MCR400 à une concentration de 100µg/mL en oxyde de fer sont mis en présence de cellules U87-Luc et de macrophages dans leur milieu de culture respectif DMEM et RPMI avec 10% de sérum de veau foetal decomplémenté, à 37°C pendant 2 heures. Les cellules U87-Luc ont été lavées cinq fois avec du PBS (phosphate buffer saline) puis fixées, inclus dans la résine et imagées par MET (microscopie électronique à transmission). Les macrophages ont été lavés cinq fois avec du PBS puis imagés par microscopie optique à épi-fluorescence. Dans les deux cas, l'internalisation des MCR400 dans les lysosomes a été observée. Lors de l'internalisation des magnétosomes dans les lysosomes, le pH s'acidifierait à une valeur typiquement comprise entre 3,5 et 5, ce qui pourrait dissocier la rhodamine B des magnétosomes.

Une suspension de MCR400 à 400 µg /mL en oxyde de fer et une solution de rhodamine B à 125µM ont été mélangées avec des solutions d'acide chlorhydrique ou avec des solutions d'hydroxyde de sodium à des concentrations variant de 0,1 à 12 M, pour ajuster le pH à des valeurs comprises entre 2 et 12. Les échantillons contenant les MCR400 ainsi traités sont ensuite placés contre un aimant de 0,6 T, pendant 12h à 4°C et le surnageant des suspensions de MCR400 traitées est retiré. L'intensité maximale de luminescence du surnageant des MCR400, excitée à 550 nm, diminue de 140 u. a. à pH 2 à 20 u. a. à pH 12. Par contre, l'intensité maximale de luminescence de la rhodamine B seule, excitée à 550 nm, augmente de 440 u. a. à 580 u. a. Cette différence de comportement suggère que la rhodamine B associée aux MCR400 subit lors d'une variation de pH une transformation telle qu'une dissociation ou modification chimique qui est différente de celle de la rhodamine B. Par ailleurs, la longueur d'onde d'émission des MCR400 est comprise entre 568 nm et 576 nm pour pH > 4, des valeurs plus faibles que les longueurs d'onde d'émission de la rhodamine B seule et du surnageant des MCR400, comprises entre 577 nm et 580 nm pour pH >4. En revanche, pour 2<pH<4, les longueurs d'onde d'émission de la rhodamine B seule, des MCR400 et du surnageant des MCR400 sont similaires, comprises entre 580 nm et 584 nm. Ces comportements pourraient s'expliquer par l'association de la rhodamine B aux magnétosomes pour pH>4 et par la dissociation de la rhodamine B des magnétosomes pour pH<4.

### Nous pouvons conclure que :

(i) Il est possible de détecter une variation de luminescence des MCR400 à l'intérieur de tumeurs de rats. Cette variation de luminescence pourrait être attribuée à une variation de pH induite par l'internalisation des MCR400 dans les cellules, en particulier à l'intérieur d'organelles à pH acide, telles que des lysosomes ou endosomes, comme nous l'avons observé par microscopie à transmission électronique.
(ii) Cette variation de luminescence des MCR400 en fonction du pH pourrait être attribuée à la dissociation de la rhodamine B des magnétosomes à pH acide.

### Exemple 7 : Utilisation des MCR400, BNF-Dil et MC-Dil comme sonde de température et de champ magnétique.

### Matériel et méthodes :

Pour les expériences réalisées sur des cerveaux de souris, les MCR400 sont introduits dans des cerveaux extraits de souris. Dans ce cas, 2 µl ou 20 µl d'une suspension de MCR400 de concentration 20 mg/mL en maghémite sont introduits à une profondeur de 1 mm dans le cerveau.

Pour les expériences réalisées dans des tissus broyés, les magnétosomes MCR400 sont mélangés à du tissu de cerveau, préalablement broyé. Dans ce cas, 2 µl ou 20 µl d'une suspension de MCR400 de concentration 20 mg/mL en maghémite sont mélangés à du tissu de cerveau contenu dans un volume de 2 mm³.

La fluorescence des MCR400 introduits dans le cerveau de souris ou mélangés au tissu est ensuite excitée à 405 nm à l'aide d'une diode laser et est détectée par un spectromètre de luminescence fibré décrit dans l'exemple 6. La fibre optique est positionnée à une distance de 3 mm au-dessus de la surface du cerveau ou du tissu broyé. Cette distance a été optimisée car elle permet de détecter l'intensité de fluorescence maximale émise par les MCR400. La fluorescence des MCR400 est mesurée soit en absence d'application d'un champ magnétique alternatif soit en présence d'un champ magnétique alternatif de fréquence 198 kHz et d'intensité 8 mT ou 25 mT. L'application du champ magnétique alternatif produit une excitation magnétique des MCR400. La température de la zone où se situent les MCR400 est également mesurée à l'aide d'une caméra infra-rouge thermographique Easir 2 et d'un thermocouple microprobe (IT-18, Physitemp, Clifton, USA).

Pour les expériences réalisées dans l'eau, 300 µl de trois suspensions contenant soit des MCR400, soit des MC-Dil, soit des BNF-Dil à une concentration de 3,6 mg/mL en maghémite sont placés à l'intérieur d'un tube et soumis à l'application d'un champ magnétique alternatif d'intensité 25 mT et de fréquence 198 kHz pendant différentes durées de 0, 100, 200, 300, 600, 900 ou 1200 secondes. La luminescence de ces suspensions est mesurée pour ces suspensions mélangées à leur surnageant, le surnageant de ces suspensions et ces suspensions mélangées dans l'eau. Les suspensions mélangées dans leur surnageant sont les suspensions obtenues après application du champ magnétique alternatif. Le surnageant de ces suspensions est obtenu en positionnant un aimant de Néodinium de 0,6 T contre la cuve en quartz contenant ces suspensions pour maintenir les différentes nanoparticules collées à la paroi de la cuve. Le surnageant est ensuite aspiré à l'aide d'une pipette. Les suspensions mélangées dans l'eau sont obtenues en retirant le surnageant des suspensions et en le remplaçant par de l'eau. La luminescence de ces trois types d'échantillon est excitée à 405 nm pour MCR400 et à 550 nm pour les BNF-DiI et les MC-Dil.

### Résultats et discussion :

Lorsque 2 µl (40 µg) ou 20 µl (400 µg) d'une suspension de MCR400 sont introduits dans le cerveau d'une souris sans application d'un champ magnétique, nous avons montré que ni l'intensité de luminescence ni la température des MCR400 ne varient au cours du temps. Un résultat similaire est obtenu lorsque les MCR400 sont soumis à l'application d'un champ magnétique alternatif de fréquence 198 kHz et d'intensité 8 mT.

En revanche, lorsque 2 µl d'une suspension de MCR400 sont introduits dans le cerveau d'une souris et les MCR400 sont soumis une première fois (passage 1) à l'application d'un champ magnétique alternatif de fréquence 198 kHz et d'intensité 25 mT, l'intensité de luminescence et la température des MCR400 augmentent de 5 unités arbitraires (ua) à 30 ua, c'est-à-dire de 80%, et de 13 °C à 14 °C respectivement pendant les 100 première secondes de l'application du champ. Après un premier traitement magnétique de 30 minutes (passage 1), les MCR400 sont soumis à 4 autres excitations magnétiques successives (passages 2 à 5). Pour les passages 2 à 5, les variations de luminescence sont toujours présentes mais moins prononcées que pour le passage 1. Des variations de température ne sont plus observées.

Lorsque 20 µl d'une suspension de MCR400 sont introduits dans le cerveau d'une souris et que les MCR400 sont soumis pendant 30 minutes à l'application d'un champ magnétique alternatif de fréquence 198 kHz et d'intensité moyenne 25 mT, l'intensité de luminescence et la température augmentent. Pendant les 100 premières secondes de l'excitation magnétique, l'intensité de luminescence augmente de 20 ua à 90 ua, c'est-à-dire de ~ 80% pour 20 µl de MCR400 administrés. Le pourcentage d'augmentation de luminescence, ~ 80 %, est similaire pour 2 µl et 20 µl de MCR400 administrés. Pendant les 100 premières secondes de l'excitation magnétique, la température des MCR400 augmente de 9 à 12 °C. Le pourcentage d'augmentation de température de ~ 33 % est significativement inférieur au pourcentage d'augmentation de luminescence.

Lorsque 2 µl d'une suspension de MCR400 sont mélangés à du tissu de cerveau et les MCR400 sont soumis une première fois pendant trente minutes à l'application d'un champ magnétique alternatif de fréquence 198 kHz et d'intensité 25 mT, la luminescence et la température des MCR400 augmentent pendant les 100 premières secondes de l'excitation magnétique d'un facteur 30 et de 2 °C respectivement, deux augmentations plus importantes que celles observées dans le cerveau.

Lorsque 300 µl d'une suspension à 3,6 mg/ml en maghémite de MCR-400, BNF-Dil et MC-Dil sont soumis à l'application d'un champ magnétique d'intensité 25 mT et de fréquence 198 kHz pendant différentes durées de 0, 100, 200, 300, 600, 900 et 1200 secondes, on observe une forte augmentation de la luminescence du surnageant durant les 100 premières secondes de l'excitation magnétique, de 1 u. a. à 19 u. a. pour le surnageant des MCR400, de 0 u. a. à 0,32 u. a. pour le surnageant des BNF-Dil et de 0 u. a. à 0,2 u. a. pour le surnageant des MC-Dil, puis la luminescence est relativement stable après 100 secondes à 18,5-19,5 u. a. pour le surnageant des MCR-400, à 0,31-0,33 u. a. pour celui des BNF-Dil et à 0,17-0,2 u. a. pour celui des MC-Dil. Contrairement à la luminescence du surnagent qui ne contient pas de particules, la luminescence des MCR-400, BNF-Dil et MC-Dil mélangés à leur surnageant ou à de l'eau reste stable à une valeur faible de ~ 4 pour les MCR400 ou reste nulle pour les BNF-Dil et les MC-Dil lorsque le champ magnétique alternatif d'intensité 25 mT et de fréquence 198 kHz est appliqué pendant différentes durées de 0, 100, 200, 300, 600, 900 et 1200 secondes.

Nous avons également mesuré la pente à l'origine de la variation de la luminescence des MCR400 introduits dans des cerveaux de souris ou mélangés à du tissu, ΔF/δt, en fonction de la pente à l'origine de la variation de température, ΔT/δt. Plus ΔT/δt augmente, plus ΔF/δt augmente et une relation proche d'une relation linéaire, peut être établie entre ΔF/δt et ΔT/δt avec un coefficient (ΔF/δt)/(ΔT/δt) ~ 45 ua/°C. Par ailleurs, lorsque ΔT/δt ~ 0, une variation de fluorescence non nulle peut être détectée, ΔF/δt ~ 0,03.

### Nous pouvons conclure de cet exemple que :

(i) lorsque l'intensité du champ magnétique alternatif est en-dessous d'un certain seuil (B < 8 mT), celui-ci ne produit ni une variation de luminescence ni une variation de température des MCR400. L'excitation magnétique des MCR400 résultant en une variation de luminescence et/ou de température nécessite l'application d'un champ magnétique alternatif d'intensité supérieure à 8 mT.
(ii) lorsque les MCR400 sont introduits dans un cerveau ou mélangés à du tissu et exposés à un champ magnétique alternatif, d'intensité supérieure à ~ 25 mT, cela peut induire une augmentation de luminescence et de température. La variation de la luminescence est plus prononcée que celle de la température. Par ailleurs, la variation de luminescence peut être détectée dans des conditions où la variation de température ne peut pas être détectée, notamment à des concentrations faibles en nanoparticules d'oxyde de fer. Cela fait de cette sonde luminescente une sonde locale plus sensible qu'une sonde macroscopique de température qui mesure une température moyenne sur un plus grand volume.
(iii) L'augmentation de la luminescence des MCR400 induite par l'application d'un champ magnétique alternatif pourrait être associée à la dissociation de la rhodamine B des magnétosomes. Celle-ci serait initialement atténuée lorsqu'elle est associée aux nanoparticules d'oxyde de fer et augmenterait lorsque la rhodamine B se dissocie des MCR400, suite à l'application du champ magnétique alternatif. Ensuite, la rhodamine B diffuserait dans l'environnement de la sonde, ce qui pourrait expliquer les variations parfois aléatoires de l'intensité de luminescence observées après 100 secondes.
(iv) L'augmentation de luminescence du surnageant des MCR400 pourrait être due à une augmentation de température causée par le champ magnétique. Par contraste, l'augmentation de luminescence du surnageant des BNF-Dil n'est pas due à l'augmentation de température induite par le champ magnétique, mais à un effet autre que thermique induit par le champ magnétique.
(v) Après avoir soumis plus de deux fois 30 minutes les MCR400 à un champ magnétique alternatif d'intensité 25 mT et de fréquence 198 kHz, l'augmentation de luminescence des MCR400 produite par l'application du champ magnétique alternatif s'atténue. Cette atténuation pourrait être due à la diffusion des MCR400 sous l'effet du champ magnétique alternatif ou à la diminution du nombre de molécules de rhodamine B associées aux magnétosomes.
(vi) les variations de luminescence et de température des MCR400 induites par l'application d'un champ magnétique alternatif d'intensité 25 mT et de fréquence 198 kHz sont corrélées. Plus la variation de luminescence augmente, plus la variation de température augmente. Par ailleurs, une relation quasi-linéaire entre ΔF/δt et ΔT/δt peut être établie pour une série de conditions différentes : (i), pour différentes quantités de nanoparticules d'oxyde de fer testées (40 µg ou 400 µg), (ii) pour différents types de nanoparticules (magnétosomes et BNF), (iii) pour différents types de fluorophores (rhodamine B et Dil) et pour différents types de préparations (des MCR400 mélangés à du tissu, insérés dans le cerveau ou mélangés dans l'eau).
(vii) Afin d'établir la relation entre la variation de luminescence et la variation de température, il est possible de considérer les variations se produisant pendant les 100 à 200 premières secondes de l'excitation due au champ magnétique alternatif. En effet, après cette période initiale de 100 à 200 secondes, la variation de luminescence des MCR400 peut être aléatoire.
(viii) lorsque une suspension de MCR400, MC-Dil, BNF-Dil est soumise à l'application d'un champ magnétique alternatif d'intensité 25 mT et de fréquence 198 kHz, cela produit une augmentation de l'intensité de luminescence du surnageant observée pendant les 100 premières secondes de l'excitation magnétique. Cette augmentation de luminescence pourrait s'expliquer par la dissociation de la rhodamine B ou du Dil des magnétosomes ou des BNF. Lorsque la rhodamine B ou le Dil est libre en solution et que les nanoparticules d'oxyde de fer ont été retirées, leur luminescence augmenterait, car elle ne serait plus atténuée par les nanoparticules d'oxyde de fer.
(ix) La luminescence des MCR400, BNF-Dil et MC-Dil mélangés au surnageant ou à de l'eau ne varie pas après application d'un champ magnétique alternatif d'intensité 25 mT et de fréquence 198 kHz. Ce comportement pourrait s'expliquer par l'absorption de la lumière par les nanoparticules d'oxyde de fer.

### Exemple 8: Mesure du temps de vie de la rhodamine B et des MCR400.

Les expériences de temps de vie ont été réalisées par la méthode fréquentielle. Le montage utilise une diode laser modulable comme source d'excitation, d'un fluo-spectroscope confocal, d'une unité contrôle de l'intensificateur d'images et de la caméra, de générateurs de fréquences et d'un contrôleur du spectrographe. La fréquence de modulation a été variée de 30 à 180 MHz. La longueur d'onde d'excitation est de 445 nm. La lumière laser est modulée par un signal de radiofréquence. Un filtre interférentiel permet de supprimer la fluorescence parasite provenant de la diode laser et un filtre passe-haut est placé dans la voie de détection pour diminuer la diffusion. Un volume de 1 mL d'une suspension de MCR400 à 5 mg/mL en oxyde de fer et de la rhodamine B à une concentration de 5µM ont été placés dans un bain chauffant à sec pendant 5 min pour chauffer les suspensions à 25°C et à 45°C. 10µL d'une suspension de MCR400 à 5 mg/mL en oxyde de fer et de la rhodamine B à une concentration de 5µM ont été déposés sur une lame puis placés devant un objectif x40 pour mesurer le temps de vie de fluorescence de chaque suspension.

A 25°C, le temps de vie de fluorescence est de 4,8 ns pour les MCR400 et de 1,6 ns pour la rhodamine B. Lorsque les échantillons sont chauffés à 45°C, le temps de vie des MCR400 est de 1,6 ns et celui de la rhodamine B est de 1,3 ns.

### Nous pouvons conclure de cet exemple que :

(i) Le temps de vie de fluorescence de la rhodamine B est plus court que celui des MCR400 à 25°C et à 45°C.
(ii) La rhodamine B est bien associée aux magnétosomes dans les MCR400 car le temps de vie de fluorescence de la rhodamine B est plus court que celui des MCR400.
(iii) Le temps de vie des MCR400 diminue de façon plus significative lorsqu'on augmente la température que celui de la rhodamine B.
(iv) Le temps de vie des MCR400 à 45°C est identique à celui de la rhodamine B seule, indiquant le décrochage de la rhodamine B des magnétosomes.

### Exemple 9: Fonctionnement de la NANOF associée à un composé pharmaceutique (non revendiqué).

Le composé pharmaceutique est associé à la NANOF par une réaction chimique lors du mélange du composé et de la NANOF. La particule est ensuite envoyée vers ou insérée dans un organisme ou une partie d'un organisme, telle qu'une tumeur ou une cellule tumorale. La particule est alors exposée à un champ magnétique, tel qu'un champ magnétique alternatif, ou à une variation de pH, de température ou de composition chimique du milieu environnant la particule, ce qui induit une dissociation du composé pharmaceutique de la NANOF et/ou modification chimique de la NANOF. Sous l'effet de cette dissociation, le composé est activé et agit comme outil de diagnostic ou de traitement thérapeutique.

### Exemple 10 : Synthèse de NANOF apyrogène et luminescente, synthétisées par des bactéries magnétotactiques, couverte d'un revêtement de poly-L-lysine et de Dil (M-PLL-DiI).

Les suspensions de NANOF apyrogènes et luminescentes de M-PLL-DiI sont préparées sous hotte dans des conditions apyrogènes en utilisant les parties centrales des magnétosomes issues de MSR-1 qui sont préparées comme suit. Des bactéries MSR-1 sont d'abord cultivées à 30°C pendant 5 à 7 jours sur un gel d'agar en présence de fer et d'une faible concentration en oxygène (~0.5% O₂). Des colonies magnétiques sont prélevées et cultivées à 30°C en présence d'air pendant plusieurs jours dans un milieu de préculture sans fer contenant des sources de carbone, d'azote, de minéraux, d'éléments trace et d'extraits de levure. Les bactéries magnétotactiques issues de la préculture sont cultivées dans un fermenteur de 50 litres à 30 °C dans un milieu similaire au milieu de préculture. Pendant la croissance, le pH est maintenu à 6,8-7 par ajout d'un milieu nutritif acide contenant une source de fer et de l'air comprimé est introduit dans le milieu de culture pour favoriser la croissance bactérienne tout en gardant la concentration en oxygène inférieure à 0,2% pour permettre la synthèse des magnétosomes. Les bactéries MSR-1 issues de la fermentation sont concentrées jusqu'à une densité optique, mesurée à 565 nm (DO₅₆₅ₙₘ), de 110-120. 100 mL de ce concentrât bactérien sont ensuite mélangés à 400 mL de NaOH 5M et chauffés à 60°C pendant 1h30 à 2h00 pour lyser les bactéries. Les magnétosomes traités sont ensuite isolés des débris bactériens en plaçant un aimant de Néodinium pendant une nuit contre la paroi du récipient contenant la suspension de bactéries lysées et en remplaçant le surnageant contenant la soude et les débris bactériens par du PBS 1X. La suspension obtenue est ensuite soniquée pendant 20 secondes à 10W en présence de PBS 1 X, placée contre un aimant de Néodinium pendant 15 minutes, le surnageant est retiré et les magnétosomes traités sont resuspendus dans du PBS 1X. Cette séquence de sonication et de séparation magnétique est répétée quatre fois. Ensuite, 100 mL de la suspension obtenue est mélangée à 200 mL d'une solution contenant du Triton X-100 à 1 % et du SDS à 1%, le mélange est chauffé pendant une nuit à 50°C, est placé contre un aimant de Néodinium, le surnageant est retiré et remplacé par 80 mL de phénol à pH 8. La suspension obtenue est chauffée pendant 2 heures en soniquant à 60°C, maintenue pendant une nuit à 60°C sans sonication, placée contre un aimant, le surnageant de la suspension est retiré et remplacé par 80 mL de chloroforme. La suspension contenant le chloroforme est placée contre un aimant de Néodinium, le surnageant est retiré et le chloroforme résiduel adsorbé à la surface des magnétosomes traités est enlevé en chauffant ces magnétosomes (sans surnageant) pendant 2 heures sous hotte. Enfin les parties centrales des magnétosomes obtenues sont désorbées de la paroi en verre des tubes qui les contiennent en ajoutant 80 mL de NaOH à 1M chauffés pendant 1 heure à 60°C au bain soniquant. La suspension contenant les parties centrales des magnétosomes est placée contre un aimant de Néodinium, le surnageant est retiré et remplacé par de l'eau MilliQ stérile, la suspension est soniquée pendant 20 secondes à 10 W. Cette séquence de lavage est répétée quatre fois. La suspension contenant les parties centrales des magnétosomes est dégazée à l'azote pour éviter l'oxydation, stérilisée par auto-clavage et stockée à -80°C.

Les suspensions contenant les parties centrales des magnétosomes sont caractérisées par un taux d'endotoxines compris entre 10 et 100 UE/mg/mL, une faible stabilité et sédimentation rapide, l'absence d'un revêtement tel que cela a été observé en microscopie à transmission électronique. 10 mL d'une suspension contenant 56 mg des parties centrales des magnétosomes sont mélangés à 373 mg de poly-L-lysine hydrobromide de masse molaire 21000 g/mol, (Gmac, CAS : 25988-63-0) et à 250 µg de DiI. Le mélange est soniqué au bain soniquant à 25kHz à 30°C pendant 15 minutes. Ensuite le mélange apyrogène est placé sous agitation à 4°C sur une roue à une vitesse de 13 rotations par minutes pendant 19 heures, puis le mélange est soniqué au doigt soniquant pendant 10 minutes à 10 W avec un pulse d'une minute et un intervalle d'une minute entre pulse, à l'abri de la lumière et à 5°C. La suspension est concentrée par centrifugation à 14 000 tours par minutes pendant 30 minutes à 5°C puis lavées deux fois à l'éthanol 0,5% et quinze fois à l'eau stérile Millipore^{®}. La suspension ainsi obtenue est finalement re-suspendues dans de l'eau stérile Millipore^{®} pour obtenir une suspension de M-PLL-DiI apyrogène.

Les cellules 3T3 sont ensemencées dans des boîtes de Pétri (Ø 30 mm) à raison de 5. 10⁵ cellules par 2 mL dans un milieu de culture (DMEM) à 10 % de NCBS. Après 24 heures, elles sont rincées au PBS puis traitées pendant 24 heures avec une suspension de M-PLL-DiI (250µg/mL en fer). Après traitement, les cellules sont rincées trois fois avec du milieu de culture avec 10% NCBS puis les cellules sont photographiées à l'aide d'un microscope à épi-fluorescence. On observe l'internalisation cellulaire des MPLL-DiI par fluorescence.

### Exemples 11 : Propriétés des MCR400 comparées à celles des MC.

Des mesures au microscope à transmission électronique révèlent que les MCR400 ont une taille moyenne de 60 nm et une distribution en taille comprise entre 15 nm et 90 nm, alors que les MC ont une taille moyenne de 40 nm et une distribution en taille comprise entre 5 nm et 60 nm. Du fait de l'augmentation de la taille des magnétosomes dans les MCR400 de 50%, comparé aux MC on peut s'attendre à une augmentation de la coercivité, du rapport entre aimantation rémanente et aimantation à saturation ou de la SAR d'au moins 0,01%, 0,1%, 1%, 2%, 5%, 10%, 25%, ou 50% chez les MCR400 comparé aux MC.

## Revendications

1. Procédé *in vitro* de détection de variation de luminescence, comprenant :
a) une étape dans laquelle une perturbation physico-chimique est exercée sur une particule utilisée à titre de sonde ;
dans laquelle la perturbation physico-chimique est exercée sur la particule par un rayonnement électromagnétique, de rayons X, un rayonnement dû à un champ magnétique, une variation de pH, et/ou une variation de température ;
ladite particule comprenant au moins une nanoparticule d'oxyde de fer ferrimagnétique ou ferromagnétique associée à au moins un composé, où la nanoparticule n'est pas liée au composé par au moins un acide nucléique ou par au moins un acide aminé, dans laquelle ledit composé se dissocie de la nanoparticule d'oxyde de fer, où la dissociation du composé n'entraine pas sa destruction, suite à ladite perturbation physico-chimique exercée sur la particule, où la perturbation physico-chimique induit une dissociation de la liaison faible entre ledit composé et ladite nanoparticule, ledit composé étant une substance luminescente qui possède au moins une fonction chimique sélectionnée dans le groupe constitué des fonctions acide carboxylique, phosphorique, sulfonique, ester, amide, cétone, alcool, phénol, thiol, amine, éther, sulfure, anhydride d'acide, halogénure d'acyle, amidine, nitrile, hydropéroxyde, imine, aldéhyde, peroxyde et ayant au-moins une propriété de luminescence qui varie lors de la dissociation entre ladite nanoparticule et ledit composé ; puis
b) une étape de détection de variation de luminescence de ladite particule utilisée à titre de sonde.

2. Procédé selon la revendication 1, dans laquelle la nanoparticule d'oxyde de fer ferrimagnétique ou ferromagnétique est synthétisée par une bactérie magnétotactique et contient un taux d'endotoxine inférieur à 10³ unité d'endotoxines par mg de particule ou moins de 90% de matière carbonée ou organique provenant de la bactérie, où ce pourcentage correspond à la masse de matière carbonée ou organique provenant de la bactérie contenue dans la particule divisée par la masse totale de la particule.

3. Procédé selon l'une quelconque des revendications **1** ou **2,** dans laquelle l'intensité de luminescence de la substance luminescente associée à la nanoparticule d'oxyde de fer est atténuée par la nanoparticule d'oxyde de fer par rapport à l'intensité de luminescence de la substance luminescente seule.

4. Procédé selon la revendication **3,** dans laquelle la variation de luminescence de la substance luminescente associée à la nanoparticule d'oxyde de fer est différente de celle de la substance luminescente seule.

5. Procédé selon l'une quelconque des revendications **3** à **4,** dans laquelle la variation d'au moins une propriété de luminescence de ladite substance luminescente est une augmentation de l'intensité de luminescence de ladite substance luminescente.

6. Procédé selon l'une quelconque des revendications **1** à **5,** dans laquelle la substance luminescente est une substance sélectionnée dans le groupe constitué de :
i) Une substance capable d'émettre de la lumière lorsqu'elle a été exposée à une radiation lumineuse,
ii) Une substance fluorescente,
iii) Une substance phosphorescente,
iv) Une substance qui possède une propriété de luminescence, sélectionnée dans le groups constituée de : une longueur d'onde de luminescence, un temps de vie de luminescence, et une anisotropie de luminescence,
v) Un luminophore,
vi) Un chromophore, et
vii) Un fluorophore

7. Procédé selon l'une quelconque des revendications **1** à **6,** dans laquelle ladite substance luminescente se dissocie de ladite nanoparticule lorsqu'elle se sépare de ladite nanoparticule pour se retrouver dans l'environnement de ladite nanoparticule.

8. Procédé selon la revendication **7** ou l'environnement de la nanoparticule d'oxyde de fer ferrimagnétique ou ferromagnétique est un milieu liquide, solide ou gazeux ou au moins 1, 10, 10², 10³, 10⁶, 10¹⁰, ou 10⁴⁰ substance(s), préférentiellement une ou des substance(s) différente(s) du composé, qui entoure(nt) ou inclus(en)t la particule sur une distance mesurée à partir du centre ou de la surface extérieure de la particule de préférence supérieure à 1 m, 1 dm, 1 cm, 1 mm, 1 µm, 100 nm, ou 10 nm.

9. Procédé selon l'une quelconque des revendications **1** à **8,** dans laquelle la liaison faible a au moins une propriété sélectionnée dans le groupe constitué de :
i) elle est **caractérisée par** une constante de dissociation K_{D} qui est inférieure à 1,
ii) elle est une liaison hydrogène,
iii) elle est une liaison qui est due à des forces de Van der Waals, de London, de Debye, des forces intermoléculaires ou inter-nanoparticulaires, ou des interactions hydrophobes ou hydrophiles ou de nature électrostatique,
iv) elle est associée à l'adsorption de ladite substance luminescente sur ladite nanoparticule, et
v) elle n'est pas une liaison forte, où la liaison forte est une liaison covalente ou ionique, ou une liaison qui empêche une dissociation de ladite substance luminescente de ladite nanoparticule,
où:
K_{D} est égale au rapport entre la concentration de ladite substance luminescente lié à ladite nanoparticule, mesurée lorsque ladite particule est activée, divisée par cette même concentration, mesurée lorsque ladite particule est inactivée,
où ladite particule est inactivée lorsqu'aucune perturbation physico-chimique n'est exercée sur elle,
et
où ladite particule est activée lorsqu'une perturbation physico-chimique est exercée sur elle.

10. Procédé selon l'une quelconque des revendications **1** à **9** :
i) pour détecter, mesurer un paramètre tel que la température, le pH, un environnement chimique, ou un rayonnement,
ii) pour modifier un paramètre, tel que l'état d'une cellule ou un état physiologique
iii) pour détecter le retrait de certaines substances d'un matériau, organisme, milieu, notamment dans le cadre d'une détoxification ou pour isoler, purifier ces substances,
iv) pour suivre le mélange de certaines substances à un matériau, organisme, ou milieu,
v) pour détecter l'efficacité d'une transfection ou magnétofection,
vi) pour détecter l'internalisation de ladite particule, par exemple dans une cellule ou une organelle où cette internalisation peut être observée à partir d'une variation de luminescence caractéristique se produisant lors de cette internalisation,
et/ou
vii) pour détecter la dissociation d'une substance de ladite particule et/ou sa diffusion suite notamment à l'application d'un rayonnement ou à la variation de l'environnement de ladite particule.

## Patentansprüche

1. In-vitro-Verfahren zum Nachweis von Lumineszenzveränderungen, umfassend:
a) einen Schritt, bei dem eine physikalisch-chemische Störung auf ein als Sonde verwendetes Partikel ausgeübt wird;
wobei die physikalisch-chemische Störung auf das Partikel durch eine elektromagnetische Strahlung, Röntgenstrahlen, eine Magnetfeldstrahlung, eine Veränderung des pH-Werts und/oder eine Temperaturveränderung ausgeübt wird;
wobei das Partikel mindestens ein ferrimagnetisches oder ferromagnetisches Eisenoxid-Nanopartikel umfasst, das mit mindestens einer Verbindung assoziiert ist, wobei das Nanopartikel nicht durch mindestens eine Nukleinsäure oder durch mindestens eine Aminosäure an die Verbindung gebunden ist, wobei sich die Verbindung von dem Eisenoxid-Nanopartikel dissoziiert, wobei die Dissoziation der Verbindung infolge der auf das Partikel ausgeübten physikalisch-chemischen Störung nicht zu ihrer Zerstörung führt, wobei die physikalisch-chemische Störung eine Dissoziation der schwachen Bindung zwischen der Verbindung und dem Nanopartikel induziert, wobei die Verbindung eine lumineszierende Substanz ist, die mindestens eine chemische Funktion besitzt, die aus der Gruppe ausgewählt ist, die aus Carbon-, Phosphor-, Sulfonsäure-, Ester-, Amid-, Keton-, Alkohol-, Phenol-, Thiol-, Amin-, Ether-, Sulfid-, Säureanhydrid-, Acylhalogenid-, Amidin-, Nitril-, Hydroperoxid-, Imin-, Aldehyd-, Peroxid-Funktionen besteht und mindestens eine Lumineszenzeigenschaft aufweist, die sich bei der Dissoziation zwischen dem Nanopartikel und der Verbindung verändert; dann
b) einen Schritt des Erfassens einer Lumineszenzveränderung des als Sonde verwendeten Partikels.

2. Verfahren nach Anspruch 1, wobei das ferrimagnetische oder ferromagnetische Eisenoxid-Nanopartikel von einem magnetotaktischen Bakterium synthetisiert wird und einen Endotoxingehalt von weniger als 10³ Endotoxineinheiten pro mg Partikel oder weniger als 90 % kohlenstoffhaltiges oder organisches Material enthält, das von dem Bakterium stammt, wobei dieser Prozentsatz der Masse an kohlenstoffhaltigem oder organischem Material entspricht, das von dem Bakterium stammt, das in dem Partikel enthalten ist, geteilt durch die Gesamtmasse des Partikels.

3. Verfahren nach einem der Ansprüche **1** oder **2,** wobei die Lumineszenzintensität der mit dem Eisenoxid-Nanopartikel assoziierten lumineszierenden Substanz durch das Eisenoxid-Nanopartikel im Verhältnis zur Lumineszenzintensität der lumineszierenden Substanz allein abgeschwächt wird.

4. Verfahren nach Anspruch **3,** wobei sich die Lumineszenzveränderung der mit dem Eisenoxid-Nanopartikel assoziierten lumineszierenden Substanz von derjenigen der lumineszierenden Substanz allein unterscheidet.

5. Verfahren nach einem der Ansprüche **3** bis **4,** wobei die Veränderung mindestens einer Lumineszenzeigenschaft der lumineszierenden Substanz eine Erhöhung der Lumineszenzintensität der lumineszierenden Substanz ist.

6. Verfahren nach einem der Ansprüche **1** bis **5,** wobei die lumineszierende Substanz eine Substanz ist, die aus der Gruppe ausgewählt ist, bestehend aus:
i) einer Substanz, die imstande ist, Licht zu emittieren, wenn sie einer Lichtstrahlung ausgesetzt wurde,
ii) einer fluoreszierenden Substanz,
iii) einer phosphoreszierenden Substanz,
iv) eine Substanz, die eine Lumineszenzeigenschaft besitzt, ausgewählt aus der Gruppe, bestehend aus: einer Lumineszenzwellenlänge, einer Lumineszenzlebensdauer und einer Lumineszenzanisotropie,
v) einem Luminophor,
vi) einem Chromophor, und
vii) einem Fluorophor.

7. Verfahren nach einem der Ansprüche **1** bis **6,** wobei sich die lumineszierende Substanz von dem Nanopartikel dissoziiert, wenn sie sich von dem Nanopartikel trennt, um in die Umgebung des Nanopartikels zu gelangen.

8. Verfahren nach Anspruch **7,** wobei die Umgebung des ferrimagnetischen oder ferromagnetischen Eisenoxid-Nanopartikels ein flüssiges, festes oder gasförmiges Medium oder mindestens 1, 10², 10³, 10⁶, 10¹⁰ oder 10⁴⁰ Substanz(en) ist, vorzugsweise eine oder mehrere Substanz(en), die sich von der Verbindung unterscheidet/unterscheiden, die das Partikel über eine Strecke, gemessen vom Zentrum oder der Außenfläche des Partikels, vorzugsweise größer als 1 m, 1 dm, 1 cm, 1 mm, 1 µm, 100 nm oder 10 nm, umgibt/umgeben oder einschließt/einschließen.

9. Verfahren nach einem der Ansprüche **1** bis **8,** wobei die schwache Bindung mindestens eine Eigenschaft besitzt, die aus der Gruppe ausgewählt ist, bestehend aus:
i) sie ist durch eine Dissoziationskonstante K_{D} gekennzeichnet, die kleiner als 1 ist,
ii) sie ist eine Wasserstoffbindung,
iii) sie eine Bindung ist, die durch Van-der-Waals-, London-, Debye-, intermolekulare oder inter-nanopartikuläre Kräfte oder durch hydrophobe oder hydrophile Wechselwirkungen oder elektrostatische Natur hervorgerufen wird,
iv) sie ist mit der Adsorption der lumineszierenden Substanz an dem Nanopartikel assoziiert,
und
v) sie ist keine starke Bindung, wobei die starke Bindung eine kovalente oder ionische Bindung ist, oder eine Bindung, die eine Dissoziation der lumineszierenden Substanz von dem Nanopartikel verhindert,
wobei:
K_{D} gleich dem Verhältnis zwischen der Konzentration der an das Nanopartikel gebundenen lumineszierenden Substanz ist, gemessen, wenn das Partikel aktiviert ist, geteilt durch dieselbe Konzentration, gemessen, wenn das Partikel inaktiviert ist,
wobei das besagte Partikel inaktiviert wird, wenn keine physikalisch-chemische Störung auf es ausgeübt wird,
und
wobei das Partikel aktiviert wird, wenn eine physikalisch-chemische Störung auf es ausgeübt wird.

10. Verfahren nach einem der Ansprüche **1** bis **9:**
i) zum Nachweis, zum Messen eines Parameters wie Temperatur, pH-Wert, eine chemische Umgebung oder Strahlung,
ii) zur Veränderung eines Parameters, z. B. des Zustands einer Zelle oder eines physiologischen Zustands,
iii) zum Nachweis der Entfernung bestimmter Substanzen aus einem Material, Organismus, Medium, insbesondere im Rahmen einer Entgiftung, oder zur Isolation, zur Reinigung dieser Substanzen,
iv) zur Nachverfolgung der Mischung bestimmter Substanzen mit einem Material, Organismus oder Medium,
v) zum Nachweis der Wirksamkeit einer Transfektion oder Magnetofektion,
vi) zum Nachweis der Internalisierung des Partikels, z. B. in einer Zelle oder Organelle, wo diese Internalisierung anhand einer charakteristischen Lumineszenzveränderung, die bei dieser Internalisierung auftritt, beobachtbar ist,
und/oder
vii) zum Nachweis der Dissoziation einer Substanz aus dem Partikel und/oder seiner Diffusion infolge insbesondere der Anwendung einer Strahlung oder der Veränderung der Umgebung des Partikels.

## Claims

1. *In vitro* method for detecting luminescence variation, comprising:
a) a step in which a physico-chemical disturbance is applied on a particle used as a probe;
in which the physico-chemical disturbance is applied on the particle by electromagnetic radiation, X-rays, radiation due to a magnetic field, a pH variation, and/or a temperature variation;
said particle comprising at least one ferrimagnetic or ferromagnetic iron oxide nanoparticle associated with at least one compound, wherein the nanoparticle is not bound to the compound by at least one nucleic acid or by at least one amino acid, wherein said compound dissociates from the iron oxide nanoparticle, wherein the dissociation of the compound does not lead to its destruction, as a result of said physicochemical perturbation applied on the particle, wherein the physicochemical perturbation induces a dissociation of the weak bond between said compound and said nanoparticle, said compound being a luminescent substance that possesses at least one chemical function selected from the group consisting of carboxylic acid, phosphoric, sulfonic, ester, amide, ketone, alcohol, phenol, thiol, amine, ether, sulfide, acid anhydride, acyl halide, amidine, nitrile, hydroperoxide, imine, aldehyde, peroxide and having at least one luminescence property which varies upon dissociation between said nanoparticle and said compound; then
b) a step for detecting the luminescence variation of said particle used as a probe.

2. A method according to claim 1, wherein the ferrimagnetic or ferromagnetic iron oxide nanoparticle is synthesized by a magnetotactic bacterium and contains an endotoxin level of less than 10³ endotoxin units per mg of particle or less than 90% of carbonaceous or organic material originating from the bacterium, where this percentage corresponds to the mass of carbonaceous or organic material originating from the bacterium contained in the particle divided by the total mass of the particle.

3. A method according to any one of claims **1** or **2,** in which the luminescence intensity of the luminescent substance associated with the iron oxide nanoparticle is quenched by the iron oxide nanoparticle compared with the luminescence intensity of the luminescent substance alone.

4. A method according to claim **3,** in which the luminescence variation of the luminescent substance associated with the iron oxide nanoparticle is different from that of the luminescent substance alone.

5. A method according to any one of claims **3** to **4,** wherein the variation of at least one luminescence property of said luminescent substance is an increase in the luminescence intensity of said luminescent substance.

6. A method according to any one of claims **1** to **5,** wherein the luminescent substance is a substance selected from the group consisting of:
i) A substance capable of emitting light when exposed to light radiation,
ii) A fluorescent substance,
iii) A phosphorescent substance,
iv) A substance that possesses a luminescence property, selected from the group consisting of: luminescence wavelength, luminescence lifetime, and luminescence anisotropy,
v) A luminophore,
vi) A chromophore, and
vii) A fluorophore

7. A method according to any one of claims **1** to **6,** wherein said luminescent substance dissociates from said nanoparticle when it separates from said nanoparticle to end up in the environment of said nanoparticle.

8. A method according to claim **7** wherein the environment of the ferrimagnetic or ferromagnetic iron oxide nanoparticle is a liquid, solid or gaseous medium or at least 1, 10, 10² , 10³ , 10⁶ , 10¹⁰ , or 10⁴⁰ substance(s), preferably one or multiple substance(s) other than the compound, which surround(s) or include(s) the particle over a distance measured from the center or outer surface of the particle preferably greater than 1 m, 1 dm, 1 cm, 1 mm, 1 µm, 100 nm, or 10 nm.

9. A method according to any one of claims **1** to **8,** wherein the weak bond has at least one property selected from the group consisting of:
i) it is **characterized by** a dissociation constant K_{D} which is less than 1,
ii) it is a hydrogen bond,
iii) it is a bond due to Van der Waals, London or Debye forces, intermolecular or inter-nanoparticle forces, hydrophobic or hydrophilic interactions or electrostatic interactions,
iv) it is associated with the adsorption of said luminescent substance on said nanoparticle, and
v) it is not a strong bond, where the strong bond is a covalent or ionic bond, or a bond that prevents dissociation of said luminescent substance from said nanoparticle,
where:
K_{D} is equal to the ratio between the concentration of said luminescent substance bound to said nanoparticle, measured when said particle is activated, divided by the same concentration, measured when said particle is inactivated,
where the particle is inactivated when no physicochemical disturbance is applied on it,
and
where said particle is activated when a physico-chemical disturbance is applied on it.

10. A method according to any one of claims **1** to **9:**
i) to detect, measure a parameter such as temperature, pH, chemical environment or radiation,
ii) to modify a parameter, such as the condition of a cell or a physiological condition,
iii) to detect the removal of certain substances from a material, organism or environment, particularly in the context of detoxification, or to isolate and purify these substances,
iv) to monitor the mixing of certain substances with a material, organism or medium,
v) to detect the efficacy of transfection or magnetofection,
vi) to detect the internalization of said particle, for example in a cell or organelle where this internalization can be observed from a characteristic luminescence variation occurring during this internalization,
and/or
vii) to detect the dissociation of a substance from said particle and/or its diffusion, in particular following the application of radiation or variation in the environment of said particle.
